(19) Europäisches Patentamt
European Patent Office
Office européen des brevets

(11) Publication number: 0 296 870
A1

# (12) EUROPEAN PATENT APPLICATION

(21) Application number: 88305772.1

(51) Int. Cl.⁴: C12N 15/00 , C12P 21/00

(22) Date of filing: 24.06.88

(30) Priority: 26.06.87 US 67653

(43) Date of publication of application:
28.12.88 Bulletin 88/52

(84) Designated Contracting States:
ES GR

(71) Applicant: E.I. DU PONT DE NEMOURS AND COMPANY
Legal Department 1007 Market Street
Wilmington Delaware 19898(US)

(72) Inventor: Ellar, David John
Wainstones Cranes Lane
Kingston Cambridge, CB3 7NJ(GB)
Inventor: Ward, Elizabeth Sally
Gonville & Caius College
Cambridge, CB3 7NJ(GB)

(74) Representative: Hildyard, Edward Martin et al
Frank B. Dehn & Co. European Patent Attorneys Imperial House 15-19 Kingsway
London WC2B 6UZ(GB)

## (54) New toxin-encoding DNA fragments from Bacillus thuringiensis subsp.israelensis.

(57) Novel DNA fragments coding for insecticidal proteins and the use of such proteins in combating insects, particularly mosquitos, are described. Chimeric genes containing the novel DNA fragments and bacteria, tissues, seeds and plants incorporating the fragments are also discussed.

EP 0 296 870 A1

# New Toxin-Encoding DNA Fragments from Bacillus thuringiensis subsp. israelensis

## BACKGROUND OF THE INVENTION

### Field of the Invention

The present invention pertains to novel DNA fragments coding for insecticidal proteins and the use of such proteins in combating insects, particularly mosquitos. Most specifically, the invention relates to fragments coding for a 130 kD mosquitocidal protein produced by Bacillus thuringiensis subsp. israelensis. Chimeric genes containing the novel DNA fragments and bacteria, tissues, seeds and plants incorporating the fragments are also discussed.

### Background

Bacillus thuringiensis subsp. israelensis, Goldberg et al., Mosquito News, 37: 355-358 (1977), is a Gram-positive spore-forming organism which produces a parasporal δ-endotoxin crystal concomitant with sporulation. Somerville, Trends Biochem. Sci., 3: 108-110 (1978); Bulla et al., Crit. Rev. Microbiol., 8: 147-204 (1980). The δ-endotoxin is extremely toxic to the larvae of mosquitos and blackflies, de Barjac, CR Acad. Sci. Paris, ser D 286: 797-800 (1978), Thomas et al., J. Cell Sci., 60: 181-197 (1983), in addition to showing cytolytic activity towards erythrocytes, Thomas et al., J. Cell Sci., 60: 181-197 (1983). Purified subsp. israelensis crystalline inclusions consist of several major polypeptide species of various molecular weights including a MW 130 kD doublet (that is, two proteins each of MW 130 kD), MW 65 kD and MW 27 kD, together with other minor proteins. See Huber et al., in "Pathogenesis of Microbial Diseases", Davidson, E. W., ed., Allanheld Osmun, New Jersey, 209-234 (1981); Thomas et al., J. Cell Sci., 60: 181-197 (1983); Yamamoto et al., Curr. Microbiol., 9: 279-284 (1983). The relative contribution of these polypeptides to the overall toxicity of the subsp. israelensis crystal is of considerable interest.

Recently there have been two approaches to the elucidation of the activities of the individual polypeptide components which comprise the subsp. israelensis crystal. Several groups have reported the separation by biochemical techniques of subsp. israelensis proteins from crystal or crystal/spore mixes. See Davidson et al., Curr. Microbiol., 11: 171-174 (1984); Thomas, Ph.D. Thesis, University of Cambridge (1984); Armstrong et al., J. Bacteriol., 161, 39-46 (1985); Wu et al., FEBS Letts., 190: 232-236 (1985); Lee et al., Biochem. Biophys. Res. Commn., 126: 953-960 (1985); Hurley et al., Biochem. Biophys. Res. Commn., 126: 961-965 (1985); Cheung et al., Curr. Microbiol., 12: 121-126 (1985); Visser et al., FEBS Letts., 30: 211-214 (1985); Ibarra et al., J. Bacteriol., 165: 527-533 (1986). Using this approach, the 130 kD doublet, 65 kD and 27 kD polypeptides have been studied in the absence of other polypeptide species.

Alternatively, separation by genetic methods has now been reported in several cases. See Ward et al., FEBS Letts., 175: 377-381 (1984); Waalwijk et al., Nucleic Acid Res., 13: 8207-8216 (1985); Sekar et al., Gene, 33:151-158 (1985); Ward et al., J. Mol. Biol., 191: 1-11 (1986); Thorne et al., J. Bacteriol., 166: 801-811 (1986). The structural gene encoding the 27 kD protein has been cloned and expressed in Escherichia coli, Ward et al., FEBS Letts., 175: 377-381 (1984), Waalwijk et al., Nucleic Acid Res., 13: 8207-8216 (1985), and in two strains of Bacillus subtilis, Ward et al., J. Mol. Biol. 191: 13-22 (1986). In addition, the nucleotide sequence of this gene has been published. See Waalwijk et al., Nucleic Acid Res., 13: 8207-8216 (1985); Ward et al., J. Mol. Biol., 191: 1-11 (1986). A structural gene encoding a mosquitocidal protein of MW 72 kD has been cloned and sequenced, Thorne et al., J. Bacteriol., 166: 801-811 (1986), and shown to share some homology with the "5.3 kb" gene isolated from subsp. kurstaki HD1-Dipel, Schnepf et al., J. Biol. Chem., 260: 6264-6272 (1985). In addition, a subsp. israelensis gene encoding a mosquitocidal protein has been isolated using Bacillus megaterium as a cloning host, Sekar et al., Gene, 33: 151-158 (1985). The nucleotide sequence of this gene has been recently reported by Yamamoto et al., in "Fundamental and Applied Aspects of Invertebrate Pathology", Samson et al., eds., (Fourth International Colloquium of Invertebrate Pathology, Netherlands, 1986), and from the length of the open reading frame it appears to code for one of the two MW 130 kD doublet proteins. Recombinant B. megaterium cells harbouring this gene synthesize a polypeptide of 130 kD which reacts immunologically with antisera raised against subsp. israelensis crystal protein, Sekar, Biochem. Biophys. Research Commn., 137: 748-751 (1986).

The present invention involves the discovery of a gene which codes for a second 130 kD protein of subsp. israelensis. The protein encoded by this gene has insecticidal properties and provides another useful agent in the ongoing battle against insect pests.

## SUMMARY OF THE INVENTION

The present invention relates to novel DNA fragments which encode insecticidal proteins. Specifically, the invention relates to a DNA fragment coding for insecticidal proteins comprising a nucleotide sequence having sequence homology or substantial sequence homology with the nucleotide sequence of a novel Bacillus thuringiensis subsp. israelensis gene shown in Figure 7 which begins with bp 1 and ends with bp 4451, and insecticidal portions thereof. An insecticidal portion thereof includes, for example, a nucleotide sequence having sequence homology or substantial sequence homology with the nucleotide sequence shown in Figure 7 which begins with bp 891 and ends with bp 4430. The subject DNA fragments code for insecticidal, particularly mosquitocidal, proteins having an amino acid sequence homologous or substantially homologous to the amino acid sequence shown in Figure 7, and insecticidal portions thereof, and the present invention also pertains to these proteins. A preferred protein is one having a molecular weight of about 130 kD. Insecticidal compositions comprising an insecticidal amount of such proteins and methods for controlling insects comprising applying to the locus to be protected an insecticidal amount of such proteins are also within the ambit of the present invention.

The present invention also relates to chimeric genes capable of expression in a microorganism, particularly Escherichia coli, comprising (1) a DNA fragment containing a promotor region and (2) a DNA fragment which encodes insecticidal proteins as described above. A preferrable promotor region is one which contains the lacZ promotor. In a preferred embodiment, the chimeric gene further comprises a 3′ inverted repeat sequence. In a most preferred embodiment where E. coli is employed, the chimeric gene further comprises the 3′ inverted repeat sequence and the promotor region contains the lacZ promotor. The most preferred embodiment is illustrated in Figure 9. The invention also pertains to microorganisms, particularly E. coli, containing such chimeric genes, and the use of such chimeric genes to modify the properties or characteristics of microorganisms.

In addition, the present invention is directed to chimeric genes capable of replication and expression in a plant cell comprising (1) a DNA fragment containing a promotor region and (2) a DNA fragment which encodes insecticidal proteins as described above. The invention also pertains to plant cells containing such chimeric genes, and plant tissues, plant seeds and plants which contain such chimeric genes in one or more of their cells and the use of such chimeric genes to modify the properties or characteristics of plant cells, plant tissues, plant seeds and/or plants.

## BRIEF DESCRIPTION OF THE FIGURES

Figure 1 provides fluorographs of SDS 13% polyacrylamide gels of the $^{35}$-S- labelled polypeptides synthesised in an E. coli transcription-translation system primed with p130/P1-1 DNA. Lane 1 contains material precipitated from an in vitro reaction using preimmune serum. Lane 2 contains material precipitated from the in vitro reaction using antiserum raised against the subsp. israelensis 130 kD doublet. Lane 3 contains $^{14}$-C-labelled molecular weight standards. The sizes (in kD) of the standards are indicated on the left margin.

Figure 2 shows the strategy used to construct the complete 130 kD δ-endotoxin gene (pPC130) described by Sekar et al., Gene, 33: 151-158 (1985) and Yamamoto et al., in "Fundamental and Applied Aspects of Invertebrate Pathology", Samson et al., eds., (Fourth International Colloquium of Invertebrate Pathology, Netherlands, 1986). Darkened circular lines represent DNA from a pUC18 vector, described by Yanisch-Perron et al., Gene, 33: 103-119 (1985). The restriction fragments used as radiolabelled probes are indicated by a thick straight line below p130/p1-1.

Figure 3 provides fluorographs of SDS 13% polyacrylamide gels of the $^{35}$-S-labelled polypeptides synthesized in the E. coli transcription-translation system primed with novel pCC130 DNA and supplemented with $^{35}$-S-methionine. Lane 1 contains material precipitated from the in vitro reaction using preimmune serum. Lane 2 contains material precipitated from the in vitro reaction using antiserum raised against the subsp. israelensis doublet. Lane 3 contains $^{14}$-C-labelled molecular weight standards. The sizes (in kD) of the standards are indicated on the left margin.

Figure 4 contains a partial restriction map of the novel subsp. israelensis DNA insert of pCC130. The open reading frame (ORF) encoding the N-terminal amino acids is indicated by the darkened region.

Figure 5 (a),(b),(c) shows the novel nucleotide sequence of the 3.94 kb insert of pCC130. The amino acid sequence corresponding to the truncated open reading frame extending from nucleotide 891 is shown below the corresponding sequence. SD indicates the position of the Shine-Dalgarno sequence, Shine et al., Nature, 245: 34-38 (1975).

Figure 6 illustrates the strategy employed for the cloning of the carboxy terminus of the novel subsp. israelensis 130 kD gene. First, a radiolabelled 1.8 kb HindIII-ClaI fragment was used to isolate a 2.3 kb HindIII fragment encoding the carboxy terminus. This fragment was then used to construct pCH130, which includes the complete δ-endotoxin gene.

Figure 7 (a),(b),(c),(d) shows the novel nucleotide sequence of the 4.46 kb insert of pCH130, which constitutes the novel 130 kD δ-endotoxin gene from subsp. israelensis. The 4.46 kb insert was provided as follows: a 3.94 kb fragment from the pCC130 insert and a 0.52 kb C-terminus, the ClaI-HindIII fragment, derived from the HindIII fragment which was isolated using the radiolabelled HindIII-ClaI fragment. The amino acid sequence corresponding to the open reading frame extending from nucleotide 891 to nucleotide 4430 is shown below the corresponding sequence. A number of restriction enzyme cleavage sites are indicated above the appropriate sequence. SD denotes the position of the Shine-Dalgarno sequence, Shine et al., Nature, 245: 34-38 (1975). The three asterisks, "***", indicate the position of the stop codon, TGA (DNA equivalent of mRNA UGA).

Figure 8 provides comparisons, using matrix analysis, of the novel structural gene sequence of the pCH130 ORF with the nucleotide sequences of previously reported Bacillus thuringiensis δ-endotoxin genes. The comparisons are with the following known gene sequences: (a) pPC130, which encodes a MW 130 kD δ-endotoxin of subsp. israelensis, described by Sekar et al., Gene, 33: 151-158 (1985) and Yamamoto et al., in "Fundamental and Applied Aspects of Invertebrate Pathology", Samson et al., eds., (Fourth International Colloquium of Invertebrate Pathology, Netherlands, 1986); (b) a subsp. israelensis gene encoding a 72 kD polypeptide described by Thorne et al., J. Bacteriol., 166: 801-811 (1986); (c) a gene encoding a MW 27 kD δ-endotoxin of subsp. israelensis described by Waalwijk et al., Nucleic Acids Res., 13: 8207-8216 (1985) and Ward et al., J. Mol. Biol., 191: 1-11 (1986); and (d) a subsp. kurstaki δ-endotoxin gene encoding a lepidopteran-specific toxin described by Schnepf et al., J. Biol. Chem., 260: 6264-6272 (1985). Nucleotide numbers are shown for each gene on the corresponding margins. Length of search elements = 15; maximum number of mismatches = 3.

Figure 9 illustrates the construction of a transcriptional fusion of the novel subsp. israelensis δ-endotoxin gene with the lacZ promoter. A unique BamHI site using the synthetic oligonucleotide 5′-CTCAATTTGGATCCACTCTTTT-3′ ( ) was created at nucleotide positions 861-867. The 1.3 kb BamHI-HindIII fragment was then used in conjuction with the 2.3 kb HindIII fragment of pCH130 to construct a transcriptional fusion of the complete toxin gene with the lacZ promoter. In addition, a HindIII-ClaI fragment derived from pPC130, encoding an extragenic 3′ inverted repeat sequence was used in the construction of pBC130.

Figure 10 depicts the immunoblotting of SDS polyacrylamide gels using antisera raised against the subsp. israelensis 130 kD doublet. Lane 1 contains approximately 200 µg of protein derived from a lysate of E. coli harbouring pBC130. Lane 2 contains approximately 100 µg of the same lysate as in Lane 1. Lane 3 contains approximately 200 µg of protein derived from a lysate of E. coli harbouring pUC18. Lane 4 contains 50 µg of purified subsp. israelensis crystal protein. An arrow indicates the position of the subsp. israelensis 130 kD doublet.

## DETAILED DESCRIPTION OF THE INVENTION

In the present written description and/or claims, reference will be made to the following abbreviations, terms and phrases.

As used herein, the letter abbreviations C, G, A and T signify the nucleotides cytosine, guanine, adenine and thymine, respectively, found in DNA. The abbreviation U signifies the nucleotide uracil, found in RNA.

As used herein, amino acids are abbreviated as follows:

Ala = alanine
Asn = asparagine
Cys = cysteine
Gln = glutamine
His = histidine
Ile = isoleucine
Arg = arginine
Asp = aspartic acid
Gly = glycine
Glu = glutamic acid
Lys = lysine
Leu = leucine
Met = methionine
Pro = proline
Thr = threonine
Tyr = tyrosine
Phe = phenylalanine
Ser = serine
Trp = tryptophan
Val = valine

The abbreviations 'bp" means base pair(s), "kb" means kilobase pairs, "kD" means kilodalton, "mD" means megadalton and "MW" means molecular weight.

The phrases "DNA fragment(s) or sequence(s)-" as used herein include single and double stranded deoxyribonucleic acid and single stranded ribonucleic acid segments, as applicable.

As used herein the phrases "sequence homology" or "homologous" denote, depending upon the context, either (1) a DNA fragment having the same nucleotide sequence as another DNA fragment or (2) a protein having the same amino acid sequence as another protein.

As used herein, the phrases "substantial sequence homology" or "substantially homologous" denote, depending upon the context, either (1) a DNA fragment having a nucleotide sequence sufficiently similar to another DNA fragment that it encodes a protein with properties or characteristics similar to the protein encoded by the other DNA fragment or (2) a protein having an amino acid sequence sufficiently similar to another protein that it exhibits properties or characteristics similar to the other protein.

The term "insecticidal portions" means, depending upon the context, either (1) those segments of a DNA fragment which code for insecticidal proteins or (2) those segments of an insecticidal protein which are themselves insecticidal.

The phrase "chimeric gene" as employed herein refers to a hybrid DNA segment comprising a promotor region from one gene source fused to at least one DNA fragment coding for an insecticidal protein from another gene source.

The term "3′ inverted repeat sequence" denotes herein a DNA sequence located downstream from the 3′ end of the novel insecticide-encoding fragments of the present invention which, when transcribed into mRNA, is expected to form a stable hairpin loop structure.

The term "promotor" herein refers to a DNA fragment, generally located upstream from the 3′ end of a coding sequence, which is involved in the binding of the enzyme RNA polymerase.

The term "microorganism", as used herein, includes unicellular organisms such as prokaryotes, e.g., Cyanophyta (blue green algae), or bacteria exemplified by Escherichia; lower eukaryotes, e.g., fungi exemplified by yeast and filamentous fungi such as Neurospora and Aspergillus, or algae exemplified by Rhodophyta, Phaeophyta and Chlorophyta; protists, e.g., amoebaes, protozoa; and viruses, e.g., baculoviruses or nuclear polyhedrosis viruses.

The term "plant cell" as used herein denotes a cell from a multicellular higher plant, whether in a whole plant or in culture.

The term "plant tissue", as used herein denotes differentiated and undifferentiated plant cells, including but not limited to roots, shoots, tumor tissue, such as crown galls, pollen, etc., whether in a whole plant or in culture.

The term "insecticidal", when used alone or in combination with other terms herein, means toxic (lethal) or combative (sublethal) to insects.

The novel insecticide-encoding DNA fragments of the present invention may be obtained from the 72 mD plasmid of Bacillus thuringiensis subsp. israelensis using the techniques of genetic engineering and molecular cloning described herein and variations thereof. Suitable variations on such techniques will be readily apparent to those skilled in the art. For general references on engineering and cloning procedures, see Maniatis et al., "Molecular Cloning: A Laboratory Manual", Cold Spring Harbor (1982). A strain of Bacillus thuringiensis subsp. israelensis carrying the 72 mD plasmid has been deposited with the National Collection of Industrial and Marine Bacteria, Torry Research Station, P.O. Box 31, 135 Abbey Road, Aberdeen, Scotland AB98DG, and bears the deposit accession number NCIB 12492.

The DNA fragment of the present invention comprises a nucleotide sequence having sequence homology or substantial sequence homology with the nucleotide sequence of a novel Bacillus thuringiensis subsp. israelensis gene shown in Figure 7 which begins with bp 1 and ends with bp 4451, and insecticidal portions thereof such as from bp 891 to bp 4430 or from bp 891 to about bp 2700. Preferably, the DNA fragment is homologous to the DNA fragment in Figure 7 from bp 1 to bp 4451 or from bp 891 to bp 4430 or from bp 891 to about bp 2700.

The proteins encoded by the present DNA fragments are insecticidal, particularly mosquitocidal. Examples of the mosquitos against which the proteins are effective include, but are not limited to, Aedes aegypti, Anopheles gambiae and Culex. The control of Aedes and Anopheles mosquitos is particularly important since these mosquitos carry the potentially life-threatening diseases yellow fever and malaria, respectively, in certain areas of the world. A preferable target mosquito is Aedes aegypti.

Although the proteins may have an amino acid sequence homologous or substantially homologous to the amino acid sequence shown in Figure 7 or an insecticidal portion thereof, the preferable proteins are homologous to the amino acid sequence shown in that Figure, or insecticidal portions thereof. Contemplated insect icidal portions thereof include, for example, proteins encoded by a DNA fragment comprising a nucleotide sequence having sequence homology or substantial sequence homology with the nucleotide sequence shown in Figure 7 from bp 891 to about bp 2700. A preferred protein is one having a molecular weight of about 130 kD, most preferably one which is homologous to the amino acid sequence shown in Figure 7.

The present invention also pertains to chimeric genes capable of expression in a microorganism or a plant cell comprising (1) a DNA fragment containing a promotor region and (2) a DNA fragment which encodes an insecticidal protein as described above. The particular promotor region utilized must be recognized by the microorganism or the plant cell; that is, it must be capable of binding the RNA polymerase enzyme of the microorganism or plant cell and directing this enzyme to the transcriptional start site of the insecticide-encoding DNA fragment.

Where expression in a microorganism is desired, a preferable microorganism is Escherichia coli or blue green algae. A number of other microorganisms, particularly other prokaryotes or lower eukaryotes such as fungi may be employed. Exemplary prokaryotes include Enterobacteriaceae such as Escherichia, Erwinia, Shigella, Salmonella and Proteus; Bacillacea; Rhizobiaceae, such as Rhizobium and Agrobacterium; Spirillaceae, such as Zymomonas, Serratia, Aeromonas, Vibrio, Desulfovibrio, Spirillum; Lactobacillaceae; Pseudomonadaceae, such as Pseudomonas and Acetobacter; Azotobacteraceae; Nitrobacteraceae; and Cyanophyta (blue green algae). Illustrative eukaryotes include algae such as Rhodophyta, Phaeophyta, Chlorophyta; fungi, such as Phycomycetes and Ascomycetes, which includes yeast such as Saccharomyces and Schizosaccharomyces; Basidiomycetes yeast such as Rhodotorula, Aureobasidium and Sporobolomyces; and mycorrhizal fungi.

A preferrable promotor region where E. coli is utilized as the host is one which contains the lacZ promotor. Other promotor regions suitable for E. coli and other microorganisms herein as well as other regulatory sequences which aid in gene expression will be apparent to those skilled in the art. See, for example, Old et al., "Principles of Gene Manipulation", Carr et al., eds. (Blackwell Scientific Publications, 1985); Rosenburg et al., Ann. Rev. of Genetics, 13: 319 (1979).

In a preferred embodiment, the chimeric gene further comprises a 3′ inverted repeat sequence. As noted above, the 3′ inverted repeat sequence involves a DNA sequence located downstream from the 3′ end of the novel insecticide-encoding fragments of the present invention which, when transcribed into mRNA, is expected to form a stable hairpin loop structure. This inverted repeat sequence is added to enhance the level of expression of the insecticide-encoding Bacillus thuringiensis subsp. israelensis DNA fragment. As one skilled in the art would recognize, to form a stable loop structure, the inverted repeat DNA sequence must contain two regions in proximity to one another which share sequence homology or substantial sequence homology, but in opposite orientation. It is believed that the more stable the loop structure, the greater the protein expression enhancement effect. As would be understood by one skilled in the art, the greater the homology of the two regions in the inverted repeat and the closer in proximity these two regions are, the greater the stability of the resulting loop structure. Suitable inverted repeat sequences will be readily apparent to those skilled in the art and include, for example, the sequence 5′-TTAAAAACCATGGAGAAAGTTTTCTCCATGGTTTTTAA-3′, as well as the inverted repeat sequence found beyond the 3′ end of the subsp. kurstaki δ-endotoxin structural gene as described in Wong et al., "Molecular Biology of Microbial Differentiation", Proceedings of the Ninth International Spores Conference, Asilomar, California, September, 1984, Hoch et al., eds., American Society of Microbiology, Washington, D.C.. A preferred inverted repeat sequence is

5′-TTAAAAACCATGGAGAAAGTTTTCTCCATGGTTTTTAA-3′

As one skilled in the art would recognize, there are other DNA fragments which, when located downstream from the 3′ end of the fragments of the present invention, will also serve to enhance protein expression. On such fragment could be the polyadenylation site sequences such as those described in "Molecular Mechanisms of Protein Biosynthesis", Weissbach et al., eds., pages 562-565 (Academic Press, N.Y. 1977); Watson et al., "Recombinant DNA: A Short Course", (W. H. Freedman & Co., N.Y., 1983); and Alberts et al., "Molecular Biology of the Cell", (Garland Publishing, N.Y. 1983).

In a most preferred embodiment where E. coli is utilized, the promoter region of the chimeric gene contains the lacZ promotor and the gene further comprises a 3′ inverted repeat sequence. A most preferred embodiment is the construct illustrated in Figure 9.

Suitable plant cells for expression of the present chimeric genes, as well as promotors and other regulatory sequences which aid in such expression, are described in European Patent Applications 193,259 and 142,924 and Rosenburg et al., Ann. Rev. of Genetics, 13: 319 (1979). Other suitable plant cells and promotors will be apparent to those skilled in the art.

The invention also pertains to microorganisms and plant cells containing such chimeric genes, and to plant tissues, plant seeds and plants which contain such chimeric genes in one or more of their cells. The chimeric genes are used to modify the properties or characteristics of such microorganisms, plant cells, plant tissues, plant seeds and/or plants. General genetic engineering procedures can be utilized to effect a transformation of microorganisms and plant cells with the chimeric gene such as those described in Maniatis et al., "Molecular Cloning: A Laboratory Manual", Cold Spring Harbor (1982) and European Patent Applications 193,259 and 142,924. The modification of properties or characteristics sought is the conference of insecticidal properties or characteristics not otherwise possessed by the microorganisms, plant cells, plant tissues, plant seeds and/or plants.

Insecticidal compositions comprising an insecticidal amount of the aforementioned proteins and methods for controlling insects comprising applying to the locus to be protected an insecticidal amount of such proteins are also within the ambit of the present invention.

In use, isolated proteins or intact microorganisms or plant cells (including plant tissues, plant seeds and/or plants) containing such proteins are applied to the locus (area) to be protected. The locus to be protected includes, for example, the habitat of the insect pests or growing vegetation or an area where vegetation is to be grown. To isolate the proteins, the microorganisms or plant cells may be lysed using conventional means such as enzymatic degradation or detergents or the like, and the protein removed and purified by standard techniques such as chromatography, extraction, electrophoresis, or the like.
Alternatively, the microorganisms or plant cells may be harvested and dried or the microorganisms, plant cells, plant tissues, plant seeds and/or plants applied intact and alive.

The isolated proteins or the harvested and dried protein-containing microorganisms or plant cells may be employed alone or as compositions. Compositions may be formulated in a variety of ways, using any of a number of conventional additives, wet or dry, depending upon the particular use. Additives can include wetting agents, detergents, stabilizers, adhering agents, spreading agents or extenders. Examples of compositions include pastes, dusting powders, wettable powders, granules, baits and aerosol compositions. Other insecticides, as well as fungicides, biocides, herbicides, or fertilizers, may also be employed along with such proteins, microorganisms or plant cells, to provide additional advantages or benefits.

Such compositions can be prepared in a conventional manner. The amount of the protein or protein-containing microorganisms or plant cells employed depends upon a variety of factors, such as the pest targeted, the composition used, the type of area to which the composition is to be applied and the prevailing weather conditions. Generally, the concentration of the insecticidal protein will be at least about 0.1% of the weight of the formulation to about 100% by weight of the formulation, more often from about 0.15% to about 0.8% weight percent of the formulation.

In practice, some of the insects feed in the protected area, that is, the area where the insecticidal protein, either alone or in a composition, has been applied. Alternatively some of the insects feed on the intact and alive chimeric gene-carrying microorganisms, plant cells, plant tissues, plant seeds and/or plants. In either situation, the insect ingests some of the insecticidal protein and suffers death or damage.

The present invention is further described by the following Examples. These Examples are illustrative only and are not to be construed as limiting the scope of the invention. All molecular weights given in the Examples refer to approximate weight average molecular weight based on the estimated weight of polyacrylamide gel samples.

## Examples

### General Procedures - Materials and Methods

*E. coli* TG1 (K12, $\alpha$(lac-pro), supE, thi, hsdD5/f traD36, proA+B+, lacI$^q$, lacZ$\Delta$M15, amp$^s$), available from the Medical Research Council (MRC), Laboratory of Molecular Biology, Cambridge, England and pUC18, as described by Yanisch-Perron et al., in Gene, 33: 103-119 (1985) and available from New England Biolabs, Bethesda Research Labs, Bethesda, MD, USA, were used as cloning host and vector respectively. For DNA sequencing M13tg130 (obtained from Amersham PLC, Amersham, Buckinghamshire, England) was used.

Restriction enzymes were obtained from New England Biolabs, Bethesda Research Labs, Bethesda, MD, USA, and were used in the buffers recommended by Maniatis et al., in "Molecular Cloning; a Laboratory Manual", Cold Spring Harbour (1982). T4 DNA ligase and the Klenow fragment of DNA polymerase were also employed and are readily available from a number of sources. Calf intestinal phosphatase (obtained form BCL, The Boehringer Corp. (London) Ltd, East Sussex, England) was used to dephosphorylate vector DNA in the medium salt buffer discussed by Maniatis et al., in "Molecular Cloning; a Laboratory Manual", Cold Spring Harbour (1982). $^{35}$-S-methionine, $\alpha$-$^{32}$-P-dATP and $\alpha$-$^{35}$-S-dATP were purchased from Amersham PLC, Amersham, Buckinghamshire, England.

The dideoxynucleotide chain termination method described by Sanger et al., Proc. Natl. Acad. Sci., USA, 74: 5463-5468 (1977) and Bankier et al., in "Techniques in the Life Sciences", Vol. B508, 1-34 (1983) was used for DNA sequencing herein. Random DNA fragments were generated using a cup horn sonicator (Heat Systems-Ultrasonics, Inc., Plainfield, N.Y., USA, Model W-375). The sonicated DNA was end-repaired, followed by size fractionation and purification of the required fraction using low-gelling temperature agarose (FMC Corporation, Marine Colloids Division, Rockland, ME 04841) gel electrophoresis. End-repaired size fractionated DNA was then ligated into SmaI restricted phosphatased M13tg130.

Recombinants were grown in 2xTY media (Bankier and Barrell, 1983) containing 100 µg/ml ampicillin to mid-exponential phase and then the inducer IPTG (Sigma) was added to a concentration of 0.5 mM. Cells were harvested after 40-44 hours of growth by centrifugation, and the resulting cell pellet washed twice in distilled water before resuspension in 50 mM Tris-acetate, pH 8.0, 10% w/v sucrose, 2 mg/ml lysozyme. The suspension was incubated for 10 minutes on ice, then sonicated (Dawes Instruments, London, England) for 4-5 periods of 30 seconds at maximum intensity. Portions of the resulting lysate were used for polyacrylamide gel or toxicity analyses.

Antisera utilized herein were raised by injecting New Zealand White rabbits with subsp. israelensis 130 kD doublet isolated by gel purification from SDS polyacrylamide gels (Walker et al., in "Methods in Peptide and Protein Sequence Analysis", 257-265 (Elsevier/North Holland Biomedical Press, 1980)). The purified protein was emulsified with Freunds complete (first injection) or incomplete (subsequent injections) adjuvant prior to subcutaneous injection. Freunds complete and incomplete adjuvant was obtained from Difco Laboratories, Detroit, MI. Antisera was analysed for specificity and titre by immunoblotting (Towbin et al., Proc. Natl. Acad. Sci., USA, 76: 4350-4354 (1979)), using horseradish peroxidase conjugated anti-rabbit immunoglobulin to detect bound antibody as described by Hawkes et al., Anal. Biochem., 119: 142-147 (1982).

## Example 1

Cloning the Novel subsp. israelensis 130 kD δ-Endotoxin Gene

Purified 72 mD plasmid was isolated from Bacillus thuringiensis subsp. israelensis as described previously by Ward et al., FEBS Letts., 175: 337-381 (1984) and used as a source of subsp. israelensis DNA in the cloning experiments. PstI restricted 72 mD plasmid was ligated into PstI phosphatased pUC18, and the resulting ligation used to transform E. coli TG1 to ampicillin resistance. DNA isolated from recombinants using a Triton-lysis method (Ward et al., FEBS Letts., 175: 337-381 (1984)) was used to prime protein synthesis in an E. coli in vitro transcription-translation system (transcription-translation system available from Amersham PLC, Amersham, Buckinghamshire, England). Products from the in vitro system were analysed by immunoprecipitation (as described in Howe et al., Mol. Gen. Genet., 186: 525-530 (1982)) using antisera raised against purified israelensis 130 kD proteins, followed by SDS polyacrylamide gel electrophoresis (as described in Laemmli, Nature, 227: 680-685 (1970)) and fluorography (as described in Chamberlain, Anal. Biochem., 98:131-135 (1979)). Using this procedure, a recombinant (identified as p130/p1-1) which directed the synthesis of MW 110 kD cross-reacting polypeptides and truncated derivatives in the in vitro system was isolated. The recombinant harbored a chimera of an 8 kb PstI fragment from the 72 mD plasmid and the cloning vector pUC18. See Figure 1. Truncated derivatives are frequently observed when using this E. coli transcription-translation system, and are presumably an artefact due to aberrant initiation or termination of translation in vitro.

The p130/P1-1 DNA was then restricted with XbaI and the resulting fragments gel purified using the procedures described by Dretzen et al., Biochem., 112: 295-298 (1981). Restriction analysis indicated that the 1.8 kb and 1.54 kb XbaI fragments are derived from the 3.34 kb PstI-XbaI-XbaI fragment previously described by Sekar et al., Gene, 33: 151-158 (1985), and recently shown by sequence analysis to encode a 130 kD subsp. israelensis polypeptide, Yamamoto et al., in "Fundamental and Applied Aspects of Invertebrate Pathology", Samson et al., eds., (Fourth International Colloquium of Invertebrate Pathology, Netherlands, 1986).

In an attempt to isolate the upstream sequences corresponding to this 130 kD gene, 1.54 kb PstI-XbaI and 1.8 kb XbaI fragments obtained from the 3.34 kb PstI-XbaI fragment were radiolabelled to a specific activty of $10^8$-$10^9$ cpm/µg using the method of Feinberg et al., Anal. Biochem., 132: 6-13 (1983). A gene bank was also constructed by ligating ClaI-restricted israelensis 72 mD plasmid into ClaI-restricted phosphatased pUC18. Recombinants obtained by transforming E. coli TG1 with the resulting ligation mix were then screened for the presence of homologous genes using the radiolabelled probes as described by Grunstein et al. Proc. Natl. Acad. Sci. USA, 78: 6091-6095 (1975). See Figure 2. Recombinants harbouring a 3.5 kb ClaI insert gave a strong hybridization signal using these fragments as probes. Further analysis indicated that these inserts overlapped with the 8 kb PstI fragment of p130/P1-1. A construct containing the complete 130 kD δ-endotoxin gene was made as shown in Figure 2, and was designated pPC130.

Interestingly, during the hybridization studies of the gene bank of the ClaI restricted 72 mD plasmid, other hybridizing colonies which produced a somewhat weaker signal upon autoradiographic exposure than the colonies which contained the 3.5 kb insert were identified. These colonies were found to harbour chimeras of a 3.9 kb ClaI fragment and pUC18 (designated pCC130). Analysis of DNA extracted from one of these recombinants in the E. coli in vitro system, see Figure 3, indicated that it encoded a polypeptide of approximately 100 kD, in addition to truncated derivatives, which were immunoprecipitated by anti 130 kD doublet sera. The restriction map of pCC130 is shown in Figure 4. The nucleotide sequence of this 3.9 kb insert was determined using the dideoxynucleotide chain termination method, and an extended open reading frame (ORF) of 3.05 kb, starting at position 891, was identified. See Figure 5.

In an attempt to isolate DNA fragments encoding the carboxy terminus of the truncated δ-endotoxin gene carried by pCC130, the 1.8 kb HindIII-ClaI fragment shown in Figure 6 was gel purified, radiolabelled by the method of Feinberg et al., Anal. Biochem., 132: 6-13 (1983), and used as a gene specific probe. Hybridization studies of a gene bank constructed by ligating HindIII restricted subsp. israelensis 72 mD plasmid DNA into the cloning vector pUC18 resulted in isolation of a 2.3 kb HindIII fragment as shown in Figure 6 which extends 520 bp to the 3′ side of the ClaI site at nucleotide position 3936 in Figure 5. DNA sequencing has shown that this fragment encodes the 3′ end of the protein encoded by the truncated open reading frame of pCC130.

Using standard recombinant DNA techniques, pCH130 (shown in Figure 6) which consists of a chimera of pUC18 and an open reading frame encoding a polypeptide of MW 130 kD was constructed. Figure 7 provides the complete nucleotide sequence of the 4.46 kb insert of pCH130, which constitutes the novel 130 kD δ-endotoxin gene from subsp. israelensis. The amino acid sequence corresponding to the open reading frame extending from nucleotide 891 to nucleotide 4430 is shown below the corresponding sequence.

The in vivo expression of the MW 130 kD protein was analysed using immunoblotting of E. coli recombinants. Antisera raised against the purified subsp. israelensis 130 kD doublet was used to detect δ-endotoxin expression. E. coli recombinants harbouring pCH130 express a gene product of MW 130 kD. The level of expression was, however, extremely low.

Comparison of the novel structural gene sequence of the pCH130 ORF with the nucleotide sequences of previously reported Bacillus thuringiensis δ-endotoxin genes is provided by the matrix analyses in Figure 8. Specifically, Figure 8 (a) compares the nucleotide sequence of the pCH130 gene with the 130 kD gene subsp. israelensis pPC130 which has been described by Sekar et al., Gene, 33: 151-158 (1985) and Yamamoto et al., in "Fundamental and Applied Aspects of Invertebrate Pathology", Samson et al., eds., (Fourth International Colloquium of Invertebrate Pathology, Netherlands, 1986). Figure 8 (b) provides comparison with the subsp. israelensis 72 kD polypeptide previously described by Thorne et al., J. Bacteriol., 166: 801-811 (1986). A comparison of the nucleotide sequence of the subsp. israelensis 27 kD gene described by Waalwijk et al., Nucleic Acids Res., 13: 8207-8216 (1985) and Ward et al., J. Mol. Biol., 191: 1-11 (1986) with that of the pCH130 gene is provided in Figure 8 (c). Finally, in Figure 8 (d) the pCH130 gene is compared with the coding sequence of the subsp. kurstaki P1 δ-endotoxin gene discussed by Schnepf et al., J. Biol. Chem., 260: 6264-6272 (1985). Although some regions of homology exist with respect to certain known genes, there remain significant regions of dissimilarity.

## Example 2

### Expression of the Novel subsp. israelensis 130 kD δ-Endotoxin Gene

In an attempt to raise the in vivo expression of this protein in E. coli, a transcriptional fusion of the ORF of pCH130 with the inducible lacZ promoter of pUC18 was constructed using the strategy shown in Figure 9. Site directed mutagenesis using a synthetic oligonucleotide as described by Carter et al., in "Oligonucleotide Site-Directed Mutagenesis in M13" (Anglian Biotechnology, Ltd, Colchester, Essex, England, 1985) was employed to direct 2 base pair changes between nucleotides 862 and 867 which created a unique BamHI restriction site at this position. This modified DNA was then used to construct pBC130 as shown in Figure 3. The plasmid pBC130 contains the 130 kD δ-endotoxin gene together with the corresponding Shine-Dalgarno sequence described in Shine et al., Nature, 254: 34-38 (1975) ligated in transcriptional frame with the lacZ promoter. In addition, recent reports (see Wong et al., "Molecular Biology of Microbial Differentiation", Proceedings of the Ninth International Spores Conference, Asilomar, California, September, 1984, Hoch et al., eds., American Society of Microbiology, Washington, D.C.) have indicated that for the subsp. kurstaki δ-endotoxin gene, an inverted repeat sequence beyond the 3′ end of the subsp. kurstaki structural gene is important in enhancing the level of expression of cloned genes in both E. coli and B. subtilis. In the construction of pBC130, therefore, a 3′ inverted repeat sequence derived from the HindIII-ClaI fragment of pPC130, was ligated to the subsp. israelensis DNA beyond the ORF. Specifically, the 3′ inverted repeat sequence utilized had the following sequence:

5′-TTAAAAACCATGGAGAAAGTTTTCTCCATGGTTTTTAA-3′

The presence of the inverted repeat sequence was found to enhance the expression of this cloned δ-endotoxin gene.

Transformation of E. coli with the pBC130 plasmid was carried out using the standard procedures described in Maniatis et al., "Molecular Cloning; a Laboratory Manual", Cold Spring Harbour (1982). The E. coli cells harbouring the pBC130 were then grown in the presence of the lacZ inducer (IPTG) for 44 hours in liquid culture as described above under the General Procedures for the growth of recombinants. Lysates of harvested cells were made as described, and analysed by polyacrylamide gel electrophoresis, followed by immunoblotting. Anti 130 kD doublet δ-endotoxin sera specifically cross-reacts with a polypeptide of MW 130 kD (Figure 10).

Example 3

Mosquitocidal Activity of the Novel subsp. israelensis 130 kD δ-Endotoxin Gene

Cultures of E. coli containing pUC18 and pBC130 were grown as described under the the General Procedures for the growth of recombinants, and the lacZ promoter of the vector induced with IPTG. Lysates of the recombinant E. coli were added to 25 third instar Aedes aegypti larvae in 2 mls tap water following the procedures described previously by Thomas et al., J. Cell Sci., 60: 181-197 (1983). After 36 hours, 70-80% larvae treated with E. coli harbouring pBC130 were dead. Larvae treated with pUC18-containing E. coli cells showed no ill effects.

The minimal amount of lysate from E. coli pBC130 required to kill A. aegypti larve has not yet been determined. Based on the assumption that the δ-endotoxin antigen in the pBC130 lysates used in this assay was 1% of the total protein, then the assay contained 10 µg δ-endotoxin protein per ml. This would therefore be the concentration sufficient to achieve a 70% mortality rate with the mosquito larvae in 36 hours.

Various modifications of the invention in addition to those illustrated and described herein will be apparent to those skilled in the art, and such modifications are intended to fall within the scope of the following claims.

**Claims**

1. A DNA fragment which encodes an insecticidal protein comprising a nucleotide sequence having sequence homology or substantial sequence homology with the nucleotide sequence shown in Figure 7 which begins with bp 1 and ends with bp 4451, and insecticidal portions thereof.

2. A DNA fragment according to Claim 1 having sequence homology or substantial sequence homology with the nucleotide sequence shown in Figure 7 which begins with bp 891 and ends with bp 4430, and insecticidal portions thereof.

3. A DNA fragment according to Claim 2 having sequence homology or substantial sequence homology with the nucleotide sequence shown in Figure 7 which begins with bp 891 and ends with about bp 2700, and insecticidal portions thereof.

4. A DNA fragment comprising a nucleotide sequence which encodes an insecticidal protein having an amino acid sequence that is homologous or substantially homologous to the amino acid sequence shown in Figure 7.

5. A DNA fragment according to Claim 2 wherein the amino acid sequence of the encoded insecticidal protein is homologous or substantially homologous to the amino acid sequence shown in Figure 7.

6. A DNA fragment according to Claim 2 wherein the molecular weight of the encoded insecticidal protein is about 130 kD.

7. A DNA fragment according to Claim 4 wherein the molecular weight of the encoded insecticidal protein is about 130 kD.

8. A chimeric gene capable of expression in a microorganism comprising a DNA fragment containing a promotor region and a DNA fragment according to Claim 2.

9. A chimeric gene capable of expression in a microorganism comprising a DNA fragment containing a promotor region and a DNA fragment according to Claim 4.

10. A chimeric gene according to Claim 8 wherein the microorganism is Escherichia coli.

11. A chimeric gene according to Claim 8 wherein the microorganism is blue green algae.

12. A chimeric gene according to Claim 10 wherein the promotor region contains the lacZ promotor.

13. A chimeric gene according to Claim 8 further comprising a 3′ inverted repeat sequence.

14. A chimeric gene according to Claim 10 further comprising a 3′ inverted repeat sequence.

15. A chimeric gene according to Claim 14 wherein the promotor region contains the lacZ promotor.

16. A chimeric gene according to Claim 15 as illustrated in Figure 9.

17. A microorganism containing a chimeric gene according to Claim 8.

18. A microorganism containing a chimeric gene according to Claim 9.

19. A microorganism containing a chimeric gene according to Claim 10.

20. A microorganism containing a chimeric gene according to Claim 11.

21. A microorganism containing a chimeric gene according to Claim 12.

22. A microorganism containing a chimeric gene according to Claim 13.

23. A microorganism containing a chimeric gene according to Claim 14.

24. A microorganism containing a chimeric gene according to Claim 15.

25. A microorganism containing a chimeric gene according to Claim 16.

26. The use of a chimeric gene according to Claim 8 to modify the properties or characteristics of a microorganism.

27. A chimeric gene capable of expression in a plant cell comprising a DNA fragment containing a promotor region and a DNA fragment according to Claim 2.

28. A chimeric gene capable of expression in a plant cell comprising a DNA fragment containing a promotor region and a DNA fragment according to Claim 4.

29. A plant cell containing a chimeric gene according to Claim 27.

30. A plant cell containing a chimeric gene according to Claim 28.

31. A plant tissue which contains in one or more of its cells a chimeric gene according to Claim 27.

32. A plant tissue which contains in one or more of its cells a chimeric gene according to Claim 28.

33. A plant seed which contains in one or more of its cells a chimeric gene according to Claim 27.

34. A plant seed which contains in one or more of its cells a chimeric gene according to Claim 28.

35. A plant which contains in one or more of its cells a chimeric gene according to Claim 27.

36. A plant which contains in one or more of its cells a chimeric gene according to Claim 28.

37. The use of a chimeric gene according to Claim 27 to modify the properties or characteristics of a plant cell, a plant tissue, a plant seed or a plant.

38. An insecticidal protein comprising an amino acid sequence having sequence homology or substantial sequence homology with the amino acid sequence shown in Figure 7, and insecticidal portions thereof.

39. An insecticidal protein according to Claim 38 wherein the molecular weight of the protein is about 130 kD.

FIG. 1

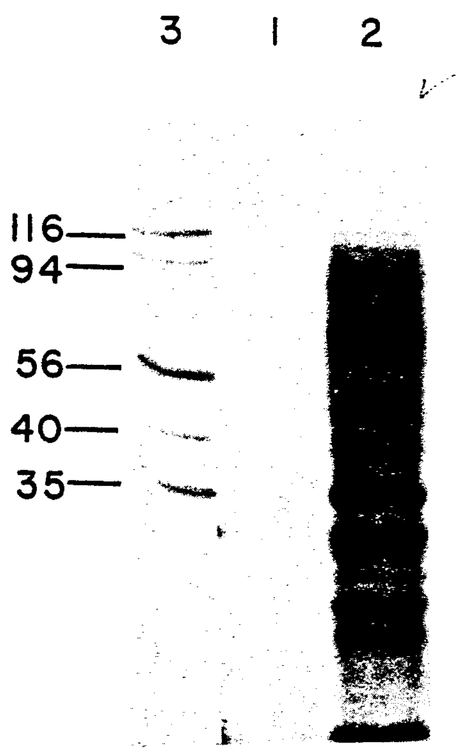


FIG. 3

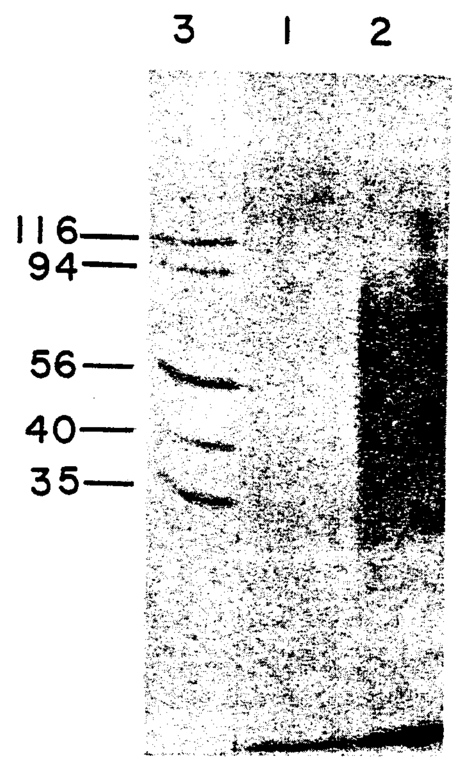


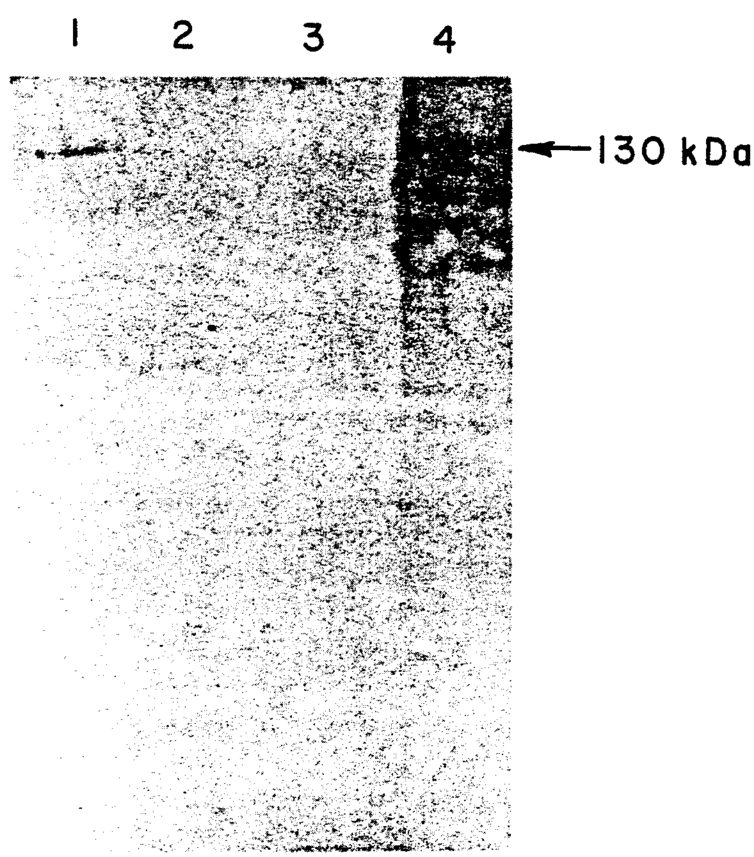


FIG. 10

FIG. 2

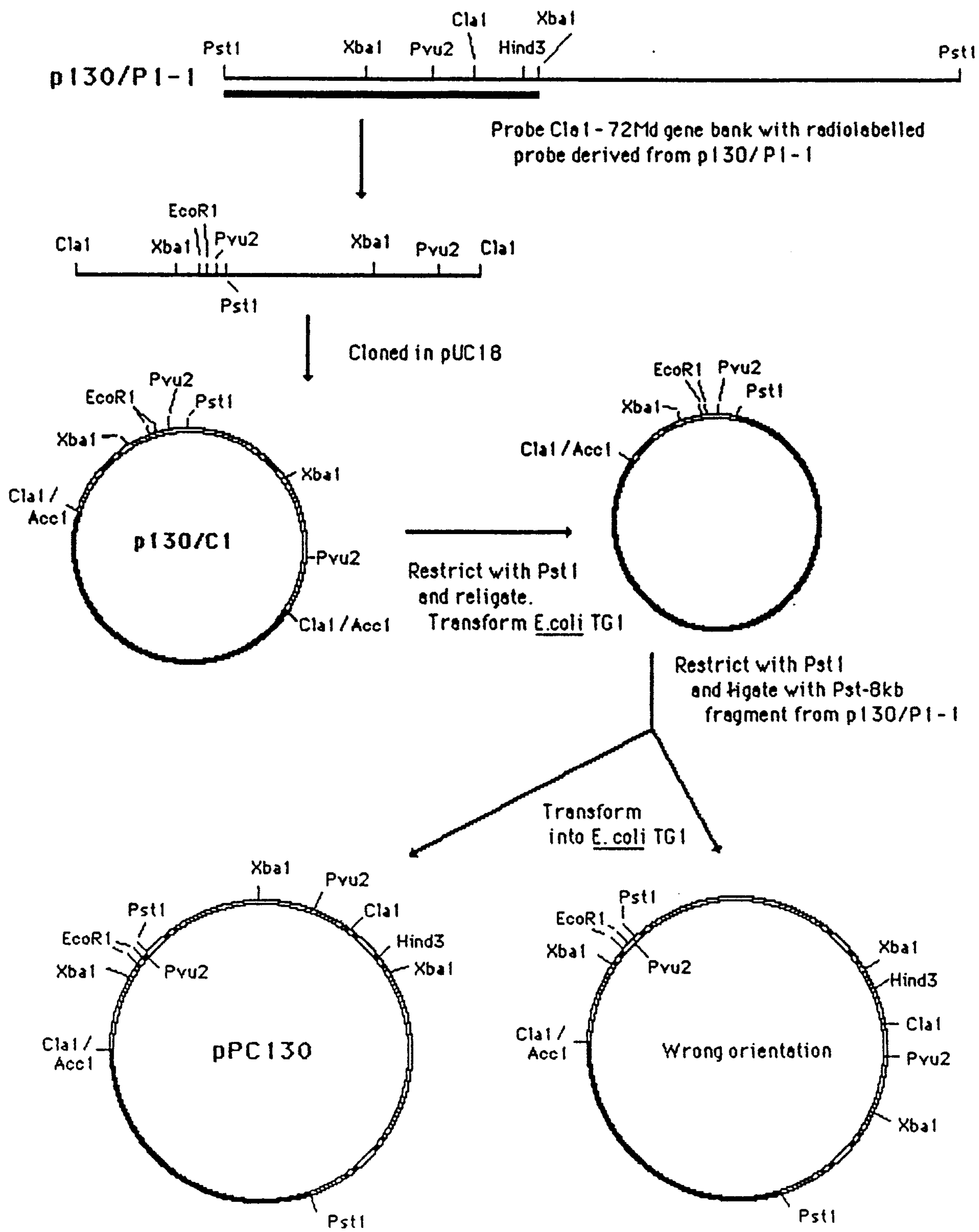
p130/P1-1
Pst1
Xba1
Pvu2
Cla1
Hind3
Xba1
Pst1
Probe Cla1-72Md gene bank with radiolabelled probe derived from p130/P1-1
Cla1
Xba1
EcoR1
Pvu2
Pst1
Xba1
Pvu2
Cla1
Cloned in pUC18
p130/C1
EcoR1
Pvu2
Pst1
Xba1
Cla1/Acc1
Xba1
Pvu2
Cla1/Acc1
Restrict with Pst1 and religate. Transform E.coli TG1
EcoR1
Pvu2
Pst1
Xba1
Cla1/Acc1
Restrict with Pst1 and ligate with Pst-8kb fragment from p130/P1-1
Transform into E. coli TG1
pPC130
Xba1
Pvu2
Cla1
Hind3
Xba1
Pst1
EcoR1
Xba1
Pvu2
Cla1/Acc1
Pst1
Wrong orientation
Pst1
EcoR1
Xba1
Pvu2
Cla1/Acc1
Xba1
Hind3
Cla1
Pvu2
Xba1
Pst1

FIG. 4

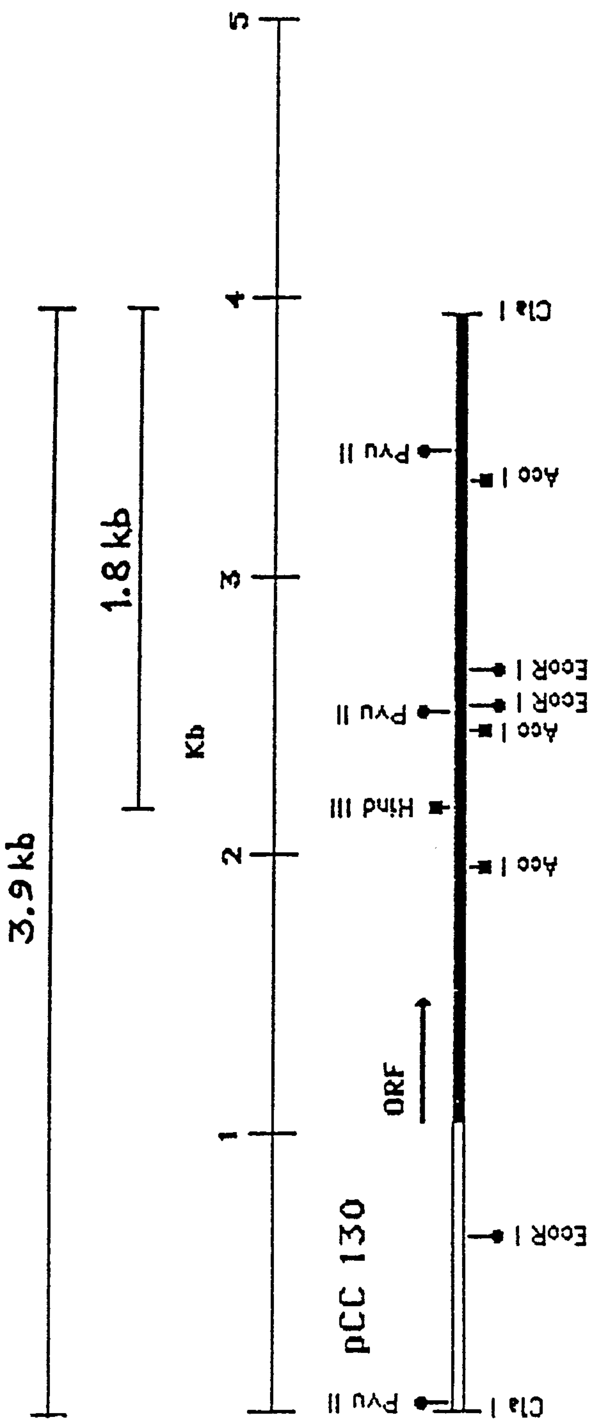
3.9 kb
1.8 kb
Kb
1
2
3
4
5
pCC 130
ORF
Pvu II
Cla I
EcoR I
Acc I
Hind III
Pvu II
EcoR I
EcoR I
Acc I
Pvu II
Cla I

# F I G. 5a

ClaI

1 TGCAGGTCGATGCAAACAGCTGTAAAAATGATTGCTGGAATAGAAGCCATGCATATGGTC

61 AAAAAAGGTCAACTCAAATTAAGGGCACAATCTGCCCAAAATCAGAATAGATGTATTCAT

121 CAGTTATTTGGATTAACTGCTTAGGAGTGGATTCCAGAAGGAATCTATGCCTTTTTTTGC

181 GTTTGTCCATTATTTGCACCAGAACCTTTGATGTTATTAAGGCGTAGAATATCCAGACAT

241 TGCAAACCAGGTGGAATGCCACGTGAGTAATTTGTATAATGAGTTTCAAATGCCTAAAAT

301 AATAAATTTACGCGAAATAATAACCTGTACGGTAGCAGTACCATAGCTTAAAAAACATCA

361 TCAGTTCCTATACTATTTGATGTAAAAGGCATATAAAGGGATATTGAAGGGGATTAGATA

421 GCTTTTTTAAAAATTAACAGGCTGGTTGATTGTCAAAAAATGACGCGGTTATTTGTTGAA

481 AGAATGTAACAGGAATAGATTATTTAAATTACGAATACTTTAAAATGCATAAGGGAAATT

541 TTGATAGCTTGGAGTGTAAAAGAAAGAGAGATTACTACATAGGAGAGGATAAATGCATAA

601 GGGTCAAAGATACCAAGAATTCTTGTAAAGAAGGTTTTCTTATTTTTGTGTGAGGTTTTG

661 TAACAAACCATATGAAATACAAAGTTTTAAAAATTTAACGCAAAAATTTTTAAAATGTAT

721 AAATTACGAATAATTATATTGGTACATTAATATGTATATAGGATTTGTAAAAGTAATCAA

781 TAATTTTCAATTTTGTACTTTTTAAACCATATCAATTTAAAAATGATTTAAAATAATCTT

841 TGTTGCTACAGAAAAAGAGTGTATCTAAATTGAGTATGGGAGGAACAAATATGAATCCT
* SD * MetAsnPro

900 TATCAAAATAAAAATGAATATGAAACATTAAATGCTTCACAAAAAAATTAAATATATCT
TyrGlnAsnLysAsnGluTyrGluThrLeuAsnAlaSerGlnLysLysLeuAsnIleSer

960 AATAATTATACAAGATATCCAATAGAAAATAGTCCAAAACAATTATTACAAAGTACAAAT
AsnAsnTyrThrArgTyrProIleGluAsnSerProLysGlnLeuLeuGlnSerThrAsn

1020 TATAAAGATTGGCTCAATATGTGTCAACAGAATCAGCAGTATGGTGGAGATTTTGAAACT
TyrLysAspTrpLeuAsnMetCysGlnGlnAsnGlnGlnTyrGlyGlyAspPheGluThr

1080 TTTATTGATAGTGGTGAACTCAGTGCCTATACTATTGTAGTTGGGACCGTACTGACTGGT
PheIleAspSerGlyGluLeuSerAlaTyrThrIleValValGlyThrValLeuThrGly

1140 TTCGGGTTCACAACACCCTTAGGACTTGCTTTAATAGGTTTTGGTACATTAATACCAGTT
PheGlyPheThrThrProLeuGlyLeuAlaLeuIleGlyPheGlyThrLeuIleProVal

1200 CTTTTTCCAGCCCAAGACCAATCTAACACATGGAGTGACTTTATAACACAAACTAAAAAT
LeuPheProAlaGlnAspGlnSerAsnThrTrpSerAspPheIleThrGlnThrLysAsn

1260 ATTATAAAAAAAGAAATAGCATCAACATATATAAGTAATGCTAATAAAATTTTAAACAGG
IleIleLysLysGluIleAlaSerThrTyrIleSerAsnAlaAsnLysIleLeuAsnArg

1320 TCGTTTAATGTTATCAGCACTTATCATAATCACCTTAAAACATGGGAGAATAATCCAAAC
SerPheAsnValIleSerThrTyrHisAsnHisLeuLysThrTrpGluAsnAsnProAsn

1380 CCACAAAATACTCAGGATGTAAGGACACAAATCCAGCTAGTTCATTACCATTTTCAAAAT
ProGlnAsnThrGlnAspValArgThrGlnIleGlnLeuValHisTyrHisPheGlnAsn

1440 GTCATTCCAGAGCTTGTAAACTCTTGTCCTCCTAATCCTAGTGATTGCGATTACTATAAC
ValIleProGluLeuVAlasnSerCysProProAsnProSerAspCysAspTyrTyrAsn

# F I G. 5b

1500 ATACTAGTATTATCTAGTTATGCACAAGCAGCAAACTTACATCTGACTGTATTAAATCAA
IleLeuValLeuSerSerTyrAlaGlnAlaAlaAsnLeuHisLeuThrValLeuAsnGln

1560 GCCGTCAAATTTGAAGCGTATTTAAAAAACAATCGACAATTCGATTATTTAGAGCCTTTG
AlaValLysPheGluAlaTyrLeuLysAsnAsnArgGlnPheAspTyrLeuGluProLeu

1620 CCAACAGCAATTGATTATTATCCAGTATTGACTAAAGCTATAGAAGATTACACTAATTAT
ProThrAlaIleAspTyrTyrProValLeuThrLysAlaIleGluAspTyrThrAsnTyr

1680 TGTGTAACAACTTATAAAAAAGGATTAAATTTAATTAAAACGACGCCTGATAGTAATCTT
CysValThrThrTyrLysLysGlyLeuAsnLeuIleLysThrThrProAspSerAsnLeu

1740 GATGGAAATATAAACTGGAACACATACAATACGTATCGAACAAAAATGACTACTGCTGTA
AspGlyAsnIleAsnTrpAsnThrTyrAsnThrTyrArgThrLysMetThrThrAlaVal

1800 TTAGATGTTGTTGCACTCTTTCCTAATTATGATGTAGGTAAATATCCAATAGGTGTCCAA
LeuAspValIleThrLeuPheProAsnTyrAspValGlyLysTyrProIleGlyValGln

1860 TCTGAACTTACTCGAGAAATTTATCAGGTACTTAACTTCGAAGAAAGCCCCTATAAATAT
SerGluLeuThrArgGluIleTyrGlnValLeuAsnPheGluGluSerProTyrLysTyr

1920 TATGACTTTCAATATCAAGAGGATTCACTTACACGTAGACCGCATTTATTTACTTGGCTT
TyrAspPheGlnTyrGlnGluAspSerLeuThrArgArgProHisLeuPheThrTrpLeu

1980 GATTCTTTGAATTTTTATGAAAAAGCGCAAACTACTCCTAATAATTTTTTCACCAGCCAT
AspSerLeuAsnPheTyrGluLysAlaGlnThrThrProAsnAsnPhePheThrSerHis

2040 TATAATATGTTTCATTACACACTTGATAATATATCCCAAAAATCTAGTGTTTTTGGAAAT
TyrAsnMetPheHisTyrThrLeuAspAsnIleSerGlnLysSerSerValPheGlyAsn

2100 CACAATGTAACTGATAAATTAAAATCTCTTGGTTTGGCAACAAATATTTATATTTTTTTA
HisAsnValThrAspLysLeuLysSerLeuGlyLeuAlaThrAsnIleTyrIlePheLeu

2160 TTAAATGTCATAAGCTTAGATAATAAATATCTAAATGATTATAATAATATTAGTAAAATG
LeuAsnValIleSerLeuAspAsnLysTyrLeuAsnAspTyrAsnAsnIleSerLysMet

2220 GATTTTTTTATAACTAATGGTACTAGACTTTTGGAGAAAGAACTTACAGCAGGATCTGGG
AspPhePheIleThrAsnGlyThrArgLeuLeuGluLysGluLeuThrAlaGlySerGly

2280 CAAATAACTTATGATGTAAATAAAAATATTTTCGGGTTACCAATTCTTAAACGAAGAGAG
GlnIleThrTyrAspVAlasnLysAsnIlePheGlyLeuProIleLeuLysArgArgGlu

2340 AATCAAGGAAACCCTACCCTTTTTCCAACATATGATAACTATAGTCATATTTTATCATTT
AsnGlnGlyAsnProThrLeuPheProThrTyrAspAsnTyrSerHisIleLeuSerPhe

2400 ATTAAAAGTCTTAGTATCCCTGCAACATATAAAACTCAAGTGTATACGTTTGCTTGGACA
IleLysSerLeuSerIleProAlaThrTyrLysThrGlnValTyrThrPheAlaTrpThr

2460 CACTCTAGTGTTGATCCTAAAAATACAATTTATACACATTTAACTACCCAAATTCCAGCT
HisSerSerVAlaspProLysAsnThrIleTyrThrHisLeuThrThrGlnIleProAla

2520 GTAAAAGCGAATTCACTTGGGACTGCTTCTAAGGTTGTTCAAGGACCTGGTCATACAGGA
ValLysAlaAsnSerLeuGlyThrAlaSerLysValValGlnGlyProGlyHisThrGly

2580 GGGGATTTAATTGATTTCAAAGATCATTTCAAAATTACATGTCAACACTCAAATTTTCAA
GlyAspLeuIleAspPheLysAspHisPheLysIleThrCysGlnHisSerAsnPheGln

2640 CAATCGTATTTTATAAGAATTCGTTATGCTTCAAATGGAAGCGCAAATACTCGAGCTGTT
GlnSerTyrPheIleArgIleArgTyrAlaSerAsnGlySerAlaAsnThrArgAlaVal

2700 ATAAATCTTAGTATCCCAGGGGTAGCAGAACTGGGTATGGCACTCAACCCCACTTTTTCT
IleAsnLeuSerIleProGlyVAlalaGluLeuGlyMetAlaLeuAsnProThrPheSer

# FIG. 5c

2760 GGTACAGATTATACGAATTTAAAATATAAAGATTTTCAGTACTTAGAATTTTCTAACGAG
GlyThrAspTyrThrAsnLeuLysTyrLysAspPheGlnTyrLeuGluPheSerAsnGlu

2820 GTGAAATTTGCTCCAAATCAAAACATATCTCTTGTGTTTAATCGTTCGGATGTATATACA
ValLysPheAlaProAsnGlnAsnIleSerLeuValPheAsnArgSerAspValTyrThr

2880 AACACAACAGTACTTATTGATAAAATTGAATTTCTGCCAATTACTCGTTCTATAAGAGAG
AsnThrThrValLeuIleAspLysIleGluPheLeuProIleThrArgSerIleArgGlu

2940 GATAGAGAGAAACAAAAATTAGAAACAGTACAACAAATAATTAATACATTTTATGCAAAT
AspArgGluLysGlnLysLeuGluThrValGlnGlnIleIleAsnThrPheTyrAlaAsn

3000 CCTATAAAAAACACTTTACAATCAGAACTTACAGATTATGACATAGATCAAGCCGCAAAT
ProIleLysAsnThrLeuGlnSerGluLeuThrAspTyrAspIleAspGlnAlaAlaAsn

3060 CTTGTGGAATGTATTTCTGAAGAATTATATCCAAAAGAAAAAATGCTGTTATTAGATGAA
LeuValGluCysIleSerGluGluLeuTyrProLysGluLysMetLeuLeuLeuAspGlu

3120 GTTAAAAATGCGAAACAACTTAGTCAATCTCGAAATGTACTTCAAAACGGGGATTTTGAA
ValLysAsnAlaLysGlnLeuSerGlnSerArgAsnValLeuGlnAsnGlyAspPheGlu

3180 TCGGCTACGCTTGGTTGGACAACAAGTGATAATATCACAATTCAAGAAGATGATCCTATT
SerAlaThrLeuGlyTrpThrThrSerAspAsnIleThrIleGlnGluAspAspProIle

3240 TTTAAAGGGCATTACCTTCATATGTCTGGGGCGAGAGACATTGATGGTACGATATTTCCG
PheLysGlyHisTyrLeuHisMetSerGlyAlaArgAspIleAspGlyThrIlePhePro

3300 ACCTATATATTCCAAAAAATTGATGAATCAAAATTAAAACCGTATACACGTTACCTAGTA
ThrTyrIlePheGlnLysIleAspGluSerLysLeuLysProTyrThrArgTyrLeuVal

3360 AGGGGATTTGTAGGAAGTAGTAAAGATGTAGAACTAGTGGTTTCACGCTATGGGGAAGAA
ArgGlyPheValGlySerSerLysAspValGluLeuValValSerArgTyrGlyGluGlu

3420 ATTGATGCCATCATGAATGTTCCAGCTGATTTAAACTATCTGTATCCTTCTACCTTTGAT
IleAspAlaIleMetAsnValProAlaAspLeuAsnTyrLeuTyrProSerThrPheAsp

3480 TGTGAAGGGTCTAATCGTTGTGAGACGTCCGCTGTGCCGGCTAACATTGGGAACACTTCT
CysGluGlySerAsnArgCysGluThrSerAlaValProAlaAsnIleGlyAsnThrSer

3540 GATATGTTGTATTCATGCCAATATGATACAGGGAAAAAGCATGTCGTATGTCAGGATTCC
AspMetLeuTyrSerCysGlnTyrAspThrGlyLysLysHisValValCysGlnAspSer

3600 CATCAATTTAGTTTCACTATTGATACAGGGGCATTAGATACAAATGAAAATATAGGGGTT
HisGlnPheSerPheThrIleAspThrGlyAlaLeuAspThrAsnGluAsnIleGlyVal

3660 TGGGTCATGTTTAAAATATCTTCTCCAGATGGATACGCATCATTAGATAATTTAGAAGTA
TrpValMetPheLysIleSerSerProAspGlyTyrAlaSerLeuAspAsnLeuGluVal

3720 ATTGAAGAAGGGCCAATAGATGGGGAAGCACTGTCACGCGTGAAACACATGGAGAAGAAA
IleGluGluGlyProIleAspGlyGluAlaLeuSerArgValLysHisMetGluLysLys

3780 TGGAACGATCAAATGGAAGCAAAACGTTCGGAAACACAACAAGCATATGATGTAGCGAAA
TrpAsnAspGlnMetGluAlaLysArgSerGluThrGlnGlnAlaTyrAspValAlaLys

3840 CAAGCCATTGATGCTTTATTCACAAATGTACAAGATGAGGCTTTACAGTTTGATACGACA
GlnAlaIleAspAlaLeuPheThrAsnValGlnAspGluAlaLeuGlnPheAspThrThr

3900 CTCGCTCAAATTCAGTACGCTGAGTATTTGGTACAATCGAT
LeuAlaGlnIleGlnTyrAlaGluTyrLeuValGlnSer

ClaI

F I G. 6

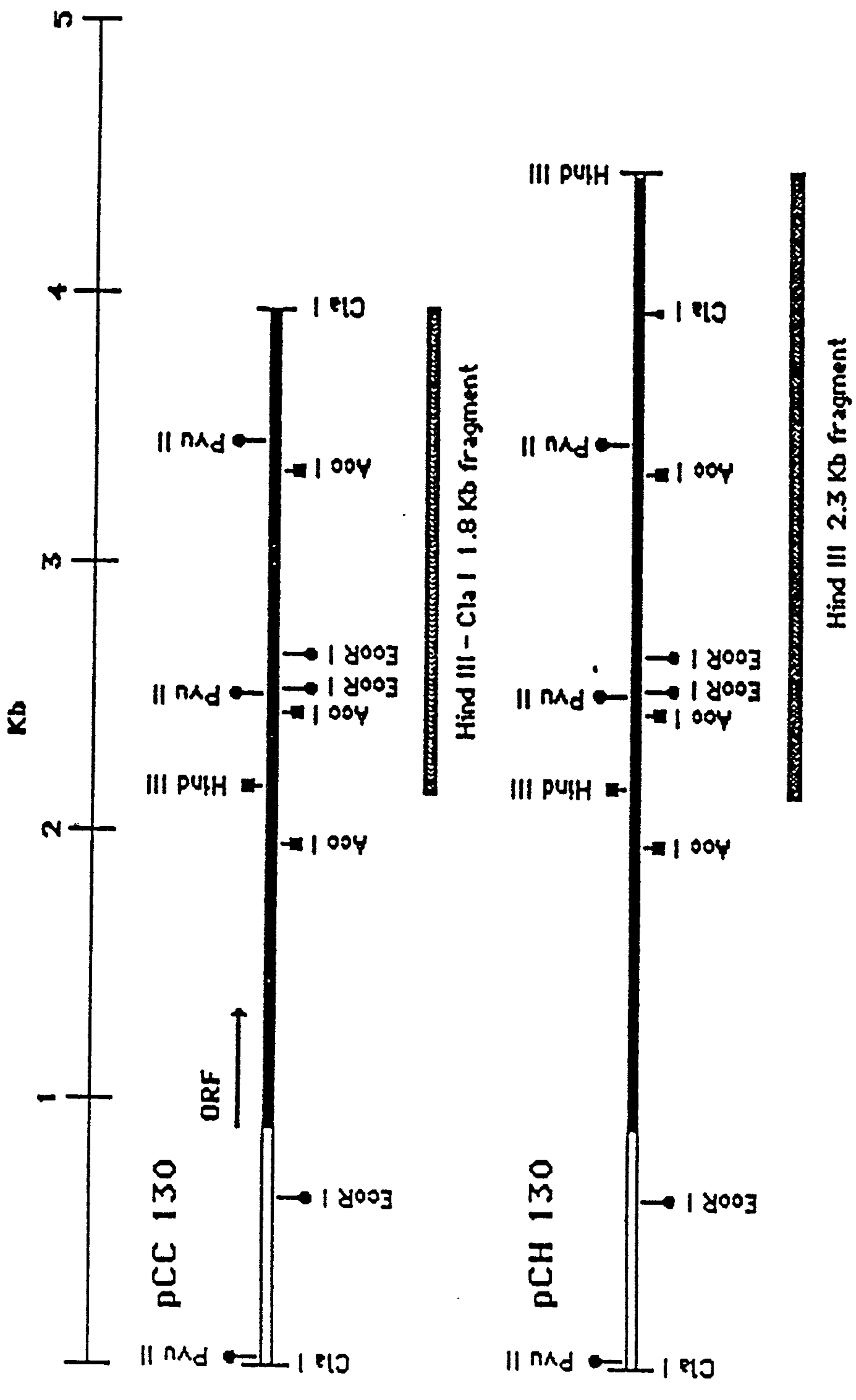
Kb
1
2
3
4
5
pCC 130
ORF
Pyu II
Cla I
EcoR I
Aco I
Hind III
Pyu II
EcoR I
EcoR I
Aco I
Pyu II
Cla I
Hind III - Cla I 1.8 Kb fragment
pCH 130
Pyu II
Cla I
EcoR I
Aco I
Hind III
Aco I
EcoR I
Pyu II
EcoR I
Aco I
Pyu II
Cla I
Hind III
Hind III 2.3 Kb fragment

# F I G. 7a

```
      ClaI               Pvu2
   1  TGCAGGTCGATGCAAACAGCTGTAAAAATGATTGCTGGAATAGAAGCCATGCATATGGTC
               Hinc2
  61  AAAAAAGGTCAACTCAAATTAAGGGCACAATCTGCCCAAAATCAGAATAGATGTATTCAT

 121  CAGTTATTTGGATTAACTGCTTAGGAGTGGATTCCAGAAGGAATCTATGCCTTTTTTTGC

 181  GTTTGTCCATTATTTGCACCAGAACCTTTGATGTTATTAAGGCGTAGAATATCCAGACAT

 241  TGCAAACCAGGTGGAATGCCACGTGAGTAATTTGTATAATGAGTTTCAAATGCCTAAAAT

 301  AATAAATTTACGCGAAATAATAACCTGTACGGTAGCAGTACCATAGCTTAAAAAACATCA

 361  TCAGTTCCTATACTATTTGATGTAAAAGGCATATAAAGGGATATTGAAGGGGATTAGATA

 421  GCTTTTTTAAAAATTAACAGGCTGGTTGATTGTCAAAAAATGACGCGGTTATTTGTTGAA

 481  AGAATGTAACAGGAATAGATTATTTAAATTACGAATACTTTAAAATGCATAAGGGAAATT

 541  TTGATAGCTTGGAGTGTAAAAGAAAGAGAGATTACTACATAGGAGAGGATAAATGCATAA
                         EcoR1
 601  GGGTCAAAGATACCAAGAATTCTTGTAAAGAAGGTTTTCTTATTTTTGTGTGAGGTTTTG

 661  TAACAAACCATATGAAATACAAAGTTTTAAAAATTTAACGCAAAAATTTTTAAAATGTAT

 721  AAATTACGAATAATTATATTGGTACATTAATATGTATATAGGATTTGTAAAAGTAATCAA

 781  TAATTTTCAATTTTGTACTTTTTAAACCATATCAATTTAAAAATGATTTAAAATAATCTT
                                BamH1
 841  TGTTGCTACAGAAAAAGAGTGTATCTAAATTGAGTATGGGAGGAACAAATATGAATCCT
                          G   C            * SD *       MetAsnPro

 900  TATCAAAATAAAAATGAATATGAAACATTAAATGCTTCACAAAAAAAATTAAATATATCT
      TyrGlnAsnLysAsnGluTyrGluThrLeuAsnAlaSerGlnLysLysLeuAsnIleSer

 960  AATAATTATACAAGATATCCAATAGAAAATAGTCCAAAACAATTATTACAAAGTACAAAT
      AsnAsnTyrThrArgTyrProIleGluAsnSerProLysGlnLeuLeuGlnSerThrAsn
                               Hinc2
1020  TATAAAGATTGGCTCAATATGTGTCAACAGAATCAGCAGTATGGTGGAGATTTTGAAACT
      TyrLysAspTrpLeuAsnMetCysGlnGlnAsnGlnGlnTyrGlyGlyAspPheGluThr

1080  TTTATTGATAGTGGTGAACTCAGTGCCTATACTATTGTAGTTGGGACCGTACTGACTGGT
      PheIleAspSerGlyGluLeuSerAlaTyrThrIleValValGlyThrValLeuThrGly

1140  TTCGGGTTCACAACACCCTTAGGACTTGCTTTAATAGGTTTTGGTACATTAATACCAGTT
      PheGlyPheThrThrProLeuGlyLeuAlaLeuIleGlyPheGlyThrLeuIleProVal

1200  CTTTTTCCAGCCCAAGACCAATCTAACACATGGAGTGACTTTATAACACAAACTAAAAAT
      LeuPheProAlaGlnAspGlnSerAsnThrTrpSerAspPheIleThrGlnThrLysAsn

1260  ATTATAAAAAAAGAAATAGCATCAACATATATAAGTAATGCTAATAAAATTTTAAACAGG
      IleIleLysLysGluIleAlaSerThrTyrIleSerAsnAlaAsnLysIleLeuAsnArg

1320  TCGTTTAATGTTATCAGCACTTATCATAATCACCTTAAAACATGGGAGAATAATCCAAAC
      SerPheAsnValIleSerThrTyrHisAsnHisLeuLysThrTrpGluAsnAsnProAsn

1380  CCACAAAATACTCAGGATGTAAGGACACAAATCCAGCTAGTTCATTACCATTTTCAAAAT
      ProGlnAsnThrGlnAspValArgThrGlnIleGlnLeuValHisTyrHisPheGlnAsn

1440  GTCATTCCAGAGCTTGTAAACTCTTGTCCTCCTAATCCTAGTGATTGCGATTACTATAAC
      ValIleProGluLeuVAlasnSerCysProProAsnProSerAspCysAspTyrTyrAsn
```

# F I G. 7 b

```
1500 ATACTAGTATTATCTAGTTATGCACAAGCAGCAAACTTACATCTGACTGTATTAAATCAA
     IleLeuValLeuSerSerTyrAlaGlnAlaAlaAsnLeuHisLeuThrValLeuAsnGln

1560 GCCGTCAAATTTGAAGCGTATTTAAAAAACAATCGACAATTCGATTATTTAGAGCCTTTG
     AlaValLysPheGluAlaTyrLeuLysAsnAsnArgGlnPheAspTyrLeuGluProLeu

1620 CCAACAGCAATTGATTATTATCCAGTATTGACTAAAGCTATAGAAGATTACACTAATTAT
     ProThrAlaIleAspTyrTyrProValLeuThrLysAlaIleGluAspTyrThrAsnTyr

1680 TGTGTAACAACTTATAAAAAAGGATTAAATTTAATTAAAACGACGCCTGATAGTAATCTT
     CysValThrThrTyrLysLysGlyLeuAsnLeuIleLysThrThrProAspSerAsnLeu

1740 GATGGAAATATAAACTGGAACACATACAATACGTATCGAACAAAAATGACTACTGCTGTA
     AspGlyAsnIleAsnTrpAsnThrTyrAsnThrTyrArgThrLysMetThrThrAlaVal

1800 TTAGATGTTGTTGCACTCTTTCCTAATTATGATGTAGGTAAATATCCAATAGGTGTCCAA
     LeuAspValIleThrLeuPheProAsnTyrAspValGlyLysTyrProIleGlyValGln

1860 TCTGAACTTACTCGAGAAATTTATCAGGTACTTAACTTCGAAGAAAGCCCCTATAAATAT
     SerGluLeuThrArgGluIleTyrGlnValLeuAsnPheGluGluSerProTyrLysTyr
                                                Acc1
1920 TATGACTTTCAATATCAAGAGGATTCACTTACACGTAGACCGCATTTATTTACTTGGCTT
     TyrAspPheGlnTyrGlnGluAspSerLeuThrArgArgProHisLeuPheThrTrpLeu

1980 GATTCTTTGAATTTTTATGAAAAAGCGCAAACTACTCCTAATAATTTTTTCACCAGCCAT
     AspSerLeuAsnPheTyrGluLysAlaGlnThrThrProAsnAsnPhePheThrSerHis

2040 TATAATATGTTTCATTACACACTTGATAATATATCCCAAAAATCTAGTGTTTTTGGAAAT
     TyrAsnMetPheHisTyrThrLeuAspAsnIleSerGlnLysSerSerValPheGlyAsn

2100 CACAATGTAACTGATAAATTAAAATCTCTTGGTTTGGCAACAAATATTTATATTTTTTTA
     HisAsnValThrAspLysLeuLysSerLeuGlyLeuAlaThrAsnIleTyrIlePheLeu
                 Hind3
2160 TTAAATGTCATAAGCTTAGATAATAAATATCTAAATGATTATAATAATATTAGTAAAATG
     LeuAsnValIleSerLeuAspAsnLysTyrLeuAsnAspTyrAsnAsnIleSerLysMet
                                                           Xho2
2220 GATTTTTTTATAACTAATGGTACTAGACTTTTGGAGAAAGAACTTACAGCAGGATCTGGG
     AspPhePheIleThrAsnGlyThrArgLeuLeuGluLysGluLeuThrAlaGlySerGly

2280 CAAATAACTTATGATGTAAATAAAAATATTTTCGGGTTACCAATTCTTAAACGAAGAGAG
     GlnIleThrTyrAspVAlasnLysAsnIlePheGlyLeuProIleLeuLysArgArgGlu

2340 AATCAAGGAAACCCTACCCTTTTTCCAACATATGATAACTATAGTCATATTTTATCATTT
     AsnGlnGlyAsnProThrLeuPheProThrTyrAspAsnTyrSerHisIleLeuSerPhe
                                                  Acc1
2400 ATTAAAAGTCTTAGTATCCCTGCAACATATAAAACTCAAGTGTATACGTTTGCTTGGACA
     IleLysSerLeuSerIleProAlaThrTyrLysThrGlnValTyrThrPheAlaTrpThr
                                                          Pvu2
2460 CACTCTAGTGTTGATCCTAAAAATACAATTTATACACATTTAACTACCCAAATTCCAGCT
     HisSerSerVAlaspProLysAsnThrIleTyrThrHisLeuThrThrGlnIleProAla
           EcoR1
2520 GTAAAAGCGAATTCACTTGGGACTGCTTCTAAGGTTGTTCAAGGACCTGGTCATACAGGA
     ValLysAlaAsnSerLeuGlyThrAlaSerLysValValGlnGlyProGlyHisThrGly
                                                 Hinc2
2580 GGGGATTTAATTGATTTCAAAGATCATTTCAAAATTACATGTCAACACTCAAATTTTCAA
     GlyAspLeuIleAspPheLysAspHisPheLysIleThrCysGlnHisSerAsnPheGln
                 EcoR1
2640 CAATCGTATTTTATAAGAATTCGTTATGCTTCAAATGGAAGCGCAAATACTCGAGCTGTT
     GlnSerTyrPheIleArgIleArgTyrAlaSerAsnGlySerAlaAsnThrArgAlaVal

2700 ATAAATCTTAGTATCCCAGGGGTAGCAGAACTGGGTATGGCACTCAACCCCACTTTTTCT
     IleAsnLeuSerIleProGlyVAlaIaGluLeuGlyMetAlaLeuAsnProThrPheSer
```

# F I G. 7c

2760 GGTACAGATTATACGAATTTAAAATATAAAGATTTTCAGTACTTAGAATTTTCTAACGAG
GlyThrAspTyrThrAsnLeuLysTyrLysAspPheGlnTyrLeuGluPheSerAsnGlu

2820 GTGAAATTTGCTCCAAATCAAAACATATCTCTTGTGTTTAATCGTTCGGATGTATATACA
ValLysPheAlaProAsnGlnAsnIleSerLeuValPheAsnArgSerAspValTyrThr

2880 AACACAACAGTACTTATTGATAAAATTGAATTTCTGCCAATTACTCGTTCTATAAGAGAG
AsnThrThrValLeuIleAspLysIleGluPheLeuProIleThrArgSerIleArgGlu

2940 GATAGAGAGAAACAAAAATTAGAAACAGTACAACAAATAATTAATACATTTTATGCAAAT
AspArgGluLysGlnLysLeuGluThrValGlnGlnIleIleAsnThrPheTyrAlaAsn

3000 CCTATAAAAAACACTTTACAATCAGAACTTACAGATTATGACATAGATCAAGCCGCAAAT
ProIleLysAsnThrLeuGlnSerGluLeuThrAspTyrAspIleAspGlnAlaAlaAsn

3060 CTTGTGGAATGTATTTCTGAAGAATTATATCCAAAAGAAAAAATGCTGTTATTAGATGAA
LeuValGluCysIleSerGluGluLeuTyrProLysGluLysMetLeuLeuleuAspGlu

3120 GTTAAAAATGCGAAACAACTTAGTCAATCTCGAAATGTACTTCAAAACGGGGATTTTGAA
ValLysAsnAlaLysGlnLeuSerGlnSerArgAsnValLeuGlnAsnGlyAspPheGlu

3180 TCGGCTACGCTTGGTTGGACAACAAGTGATAATATCACAATTCAAGAAGATGATCCTATT
SerAlaThrLeuGlyTrpThrThrSerAspAsnIleThrIleGlnGluAspAspProIle

3240 TTTAAAGGGCATTACCTTCATATGTCTGGGGCGAGAGACATTGATGGTACGATATTTCCG
PheLysGlyHisTyrLeuHisMetSerGlyAlaArgAspIleAspGlyThrIlePhePro

Acc1
3300 ACCTATATATTCCAAAAAATTGATGAATCAAAATTAAAACCGTATACACGTTACCTAGTA
ThrTyrIlePheGlnLysIleAspGluSerLysLeuLysProTyrThrArgTyrLeuVal

3360 AGGGGATTTGTAGGAAGTAGTAAAGATGTAGAACTAGTGGTTTCACGCTATGGGGAAGAA
ArgGlyPheValGlySerSerLysAspValGluLeuValValSerArgTyrGlyGluGlu

Pvu2
3420 ATTGATGCCATCATGAATGTTCCAGCTGATTTAAACTATCTGTATCCTTCTACCTTTGAT
IleAspAlaIleMetAsnValProAlaAspLeuAsnTyrLeuTyrProSerThrPheAsp

3480 TGTGAAGGGTCTAATCGTTGTGAGACGTCCGCTGTGCCGGCTAACATTGGGAACACTTCT
CysGluGlySerAsnArgCysGluThrSerAlaValProAlaAsnIleGlyAsnThrSer

3540 GATATGTTGTATTCATGCCAATATGATACAGGGAAAAAGCATGTCGTATGTCAGGATTCC
AspMetLeuTyrSerCysGlnTyrAspThrGlyLysLysHisValValCysGlnAspSer

3600 CATCAATTTAGTTTCACTATTGATACAGGGGCATTAGATACAAATGAAAATATAGGGGTT
HisGlnPheSerPheThrIleAspThrGlyAlaLeuAspThrAsnGluAsnIleGlyVal

3660 TGGGTCATGTTTAAAATATCTTCTCCAGATGGATACGCATCATTAGATAATTTAGAAGTA
TrpValMetPheLysIleSerSerProAspGlyTyrAlaSerLeuAspAsnLeuGluVal

3720 ATTGAAGAAGGGCCAATAGATGGGGAAGCACTGTCACGCGTGAAACACATGGAGAAGAAA
IleGluGluGlyProIleAspGlyGluAlaLeuSerArgValLysHisMetGluLysLys

3780 TGGAACGATCAAATGGAAGCAAAACGTTCGGAAACACAACAAGCATATGATGTAGCGAAA
TrpAsnAspGlnMetGluAlaLysArgSerGluThrGlnGlnAlaTyrAspVAlalaLys

3840 CAAGCCATTGATGCTTTATTCACAAATGTACAAGATGAGGCTTTACAGTTTGATACGACA
GlnAlaIleAspAlaLeuPheThrAsnValGlnAspGluAlaLeuGlnPheAspThrThr

Cla1
3900 CTCGCTCAAATTCAGTACGCTGAGTATTTGGTACAATCGATTCCATATGTGTACAATGAT
LeuAlaGlnIleGlnTyrAlaGluTyrLeuValGlnSerIleProTyrValTyrAsnAsp

# F I G. 7d

```
3960 TGGTTGTCAGATGTTCCAGGTATGAATTATGATATCTATGTAGAGTTGGATGCACGAGTG
     TrpLeuSerAspValProGlyMetAsnTyrAspIleTyrValGluLeuAspAlaArgVal

4020 GCACAAGCGCGTTATTTGTATGATATAAGAAATATTATTAAAAATGGTGATTTTACACAA
     AlaGlnAlaArgTyrLeuTyrAspIleArgAsnIleIleLysAsnGlyAspPheThrGln

4080 GGGGTAATGGGGTGGCATGTAACTGGAAATGCAGACGTACAACAAATAGATGGTGTTTCT
     GlyValMetGlyTrpHisValThrGlyAsnAlaAspValGlnGlnIleAspGlyValSer

4140 GTATTGGTTCTATCTAATTGGAGTGCTGGCGTATCTCAAAATGTCCATCTCCAACATAAT
     ValLeuValLeuSerAsnTrpSerAlaGlyValSerGlnAsnValHisLeuGlnHisAsn

4200 CATGGGTATGTCTTAGGTGTTATTGCCAAAAAAGAAGGACCTGGAAATGGGTATGTCACG
     HisGlyTyrValLeuGlyValIleAlaLysLysGluGlyProGlyAsnGlyTyrValThr

4260 CTTATGGATTGGGAGGAGAATCAAGAAAAATTGACGTTTACGTCTTGTGAAGAAGGATAT
     LeuMetAspTrpGluGluAsnGlnGluLysLeuThrPheThrSerCysGluGluGlyTyr

4320 ATTACGAAGACAGTAGATGTATTCCCAGATACAGATCGTGTACGAATTGAGATAGGCGAA
     IleThrLysThrValAspValPheProAspThrAspArgValArgIleGluIleGlyGlu

4380 ACCGAAGGTTCGTTTTATATCGAAAGCATTGAATTAATTTGCATGAACGAGTGATTAATA
     ThrGluGlySerPheTyrIleGluSerIleGluLeuIleCysMetAsnGlu***

                 Hind3
4440 AAAAATAACTAA
```

Neu eing… …cht / … …ly filed
Nou… …ment … …osé

FIG. 8A

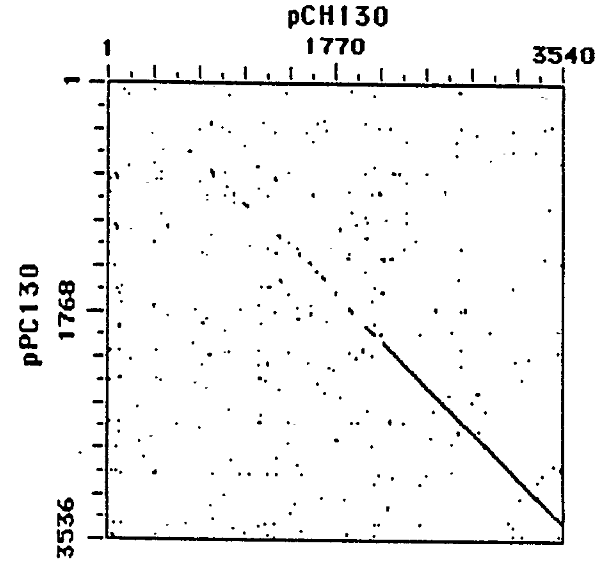


FIG. 8B

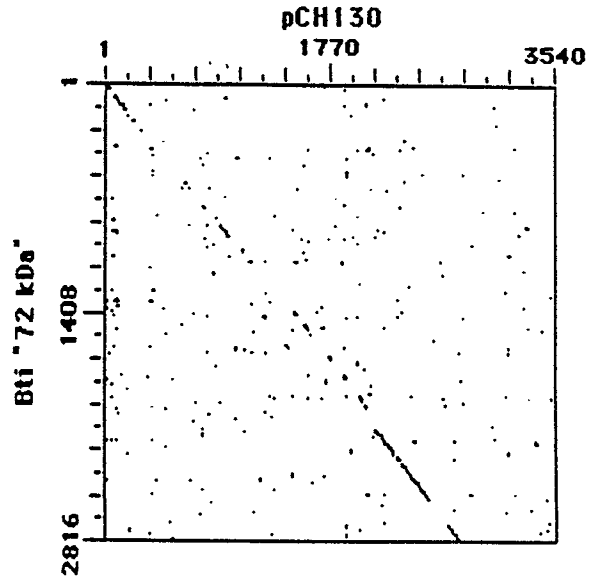


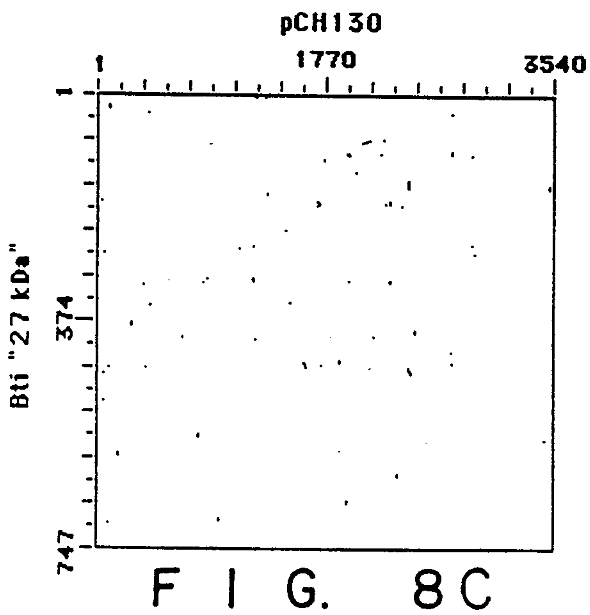


FIG. 8C

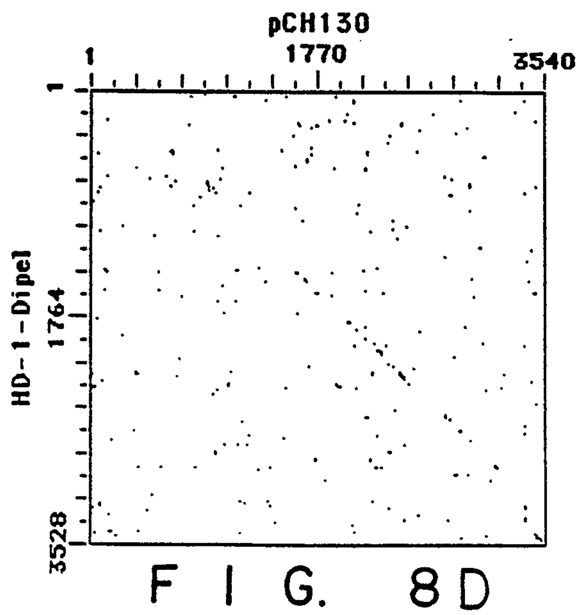


FIG. 8D

Neu ei... Newly filed
Neu ... ent déposé

F I G. 9

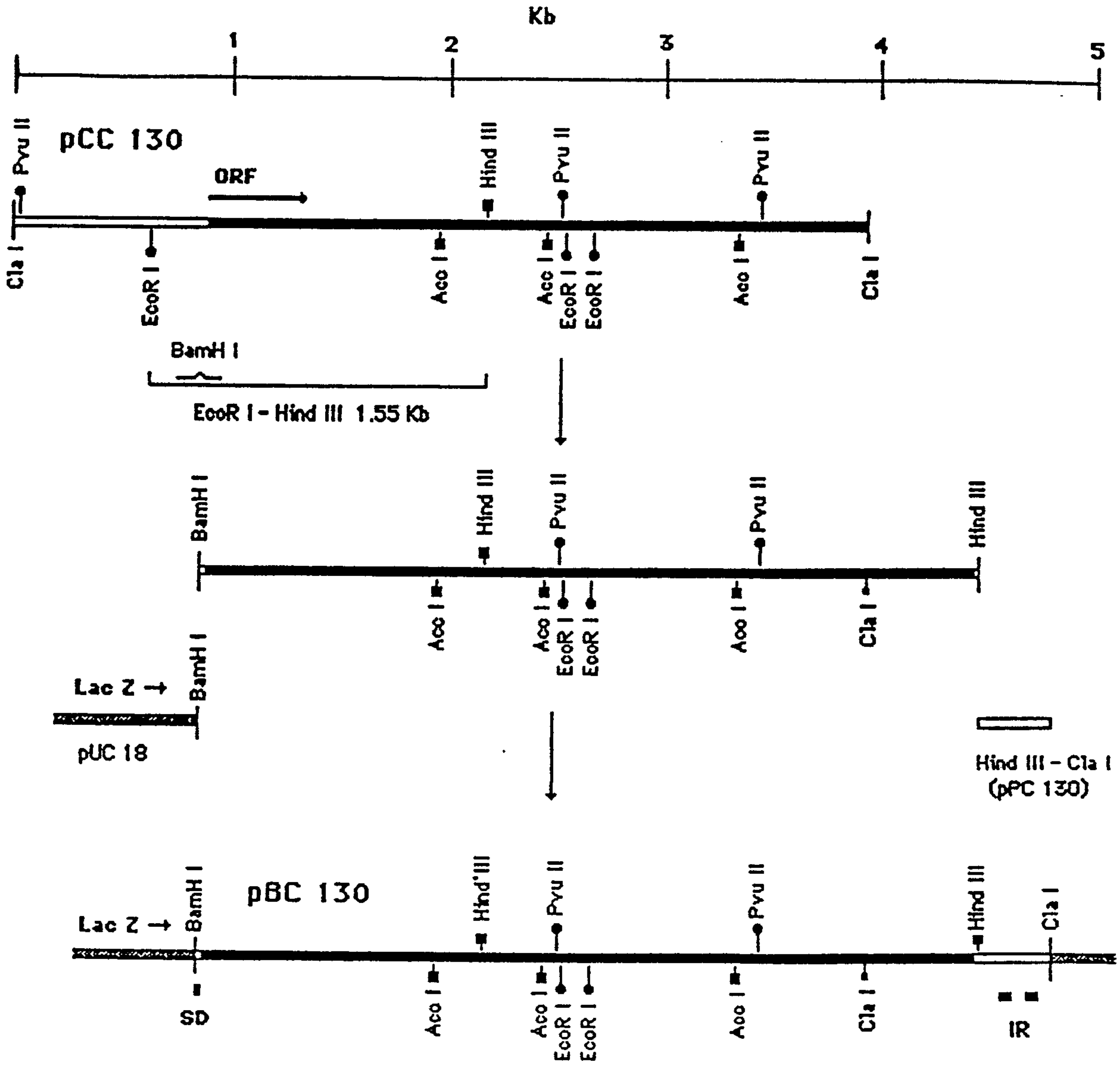

European Patent Office

# EUROPEAN SEARCH REPORT

Application Number

EP 88 30 5772

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y<br>X | EP-A-0 153 166 (SYNTRO CORP.)<br>* Whole document * | 1-37<br>38,39 | C 12 N 15/00<br>C 12 P 21/00 |
| Y | EP-A-0 195 285 (UNIVERSITY OF GEORGIA RESEARCH FOUNDATION, INC.)<br>* Claims; figurs 7-1 - 7-8; figures 8-1 - 8-2 * | 1-37 | |
| D,Y | FEBS LETTERS, vol. 175, no. 2, October 1984, pages 377-383, Federation of European Biochemical Societies, Cambridge, GB; E.S. WARD et al.: "Cloning and expression in Escherichia coli of the insecticidal delta-endotoxin gene of Bacillus thuringiensis var. israelensis",<br>* Whole article * | 1-37 | |
| Y | EP-A-0 193 259 (PLANT GENETIC SYSTEMS N.V.)<br>* Page 53, line 1 - page 61, line 22; claims * | 8-37 | **TECHNICAL FIELDS SEARCHED (Int. Cl.4)**<br>C 12 N |
| Y | WO-A-8 601 536 (WASHINGTON RESEARCH FOUNDATION)<br>* Claims 22,23 *<br>-/- | 29-37 | |

The present search report has been drawn up for all claims

| Place of search | Date of completion of the search | Examiner |
|---|---|---|
| THE HAGUE | 03-10-1988 | PULAZZINI A.F.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 88 30 5772

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | JOURNAL OF BACTERIOLOGY, vol. 166, no. 3, June 1986, pages 801-811, American Society for Microbiology, California, US; L. THORN et al.: "Structural similarity between the Lepidoptera- and Diptera-specific insecticidal endotoxin genes of Bacillus thuringiensis subsp. "kurstaki" and "israelensis" * Whole article * | 1-37 | |
| D,A | FEMS MICROBIOLOGY LETTERS, vol. 30, 1986, pages 211-214, Elsevier; B. VISSER et al.: "The mosquitocidal activity of Bacillus thuringiensis var. israelensis is associated with Mr 230 000 and 130 000 crystal proteins" | | |
| A | EP-A-0 142 924 (LUBRIZOL GENETICS, INC) | | |
| P,A | NUCLEIC ACIDS RESEARCH, vol, 15, no. 17, 11th September 1987, page 7195, IRL Press Ltd, Eynsham, Oxford, GB; E.S. WARD et al.: "Nucleotide sequence of a Bacillus thuringiensis var. israelensis gene encoding a 130 kDa deltaendotoxin" * Whole article * | | TECHNICAL FIELDS SEARCHED (Int. Cl.4) |
| P,A | NUCLEIC ACIDS RESEARCH, vol. 16, no. 4, 25th February 1988, pages 1637-38, IRL Press Ltd, Eynsham, Oxford, GB; S. TUNGPRADUBKUL et al.: "The complete nucleotide sequence of a 130 kDa mosquito-larvicidal delta-endotoxin gene of Bacillus thuringiensis var. israelensis", * Whole article * | | |

The present search report has been drawn up for all claims

| Place of search | Date of completion of the search | Examiner |
|---|---|---|
| THE HAGUE | 03-10-1988 | PULAZZINI A.F.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)